# EUROPEAN PATENT APPLICATION

(11) **EP 4 331 619 A1**
(43) Date of publication of application: **06.03.2024**
(21) Application number: 22795837.8
(22) Date of filing: 27.04.2022
(51) Int. Cl.: A61K 48/00, A61P 21/00, C07K 16/18, C12N 5/10, C12N 7/00, C12N 1/15, C12N 1/19, C12N 1/21, C12N 15/12, C12N 15/86, A61K 38/16, A61K 35/76, A61K 35/761, A61K 35/763, C07K 14/47

(54) **THERAPEUTIC DRUG FOR MYOTONIC DYSTROPHY TYPE 1**

(30) Priority: 30.04.2021 JP 2021077262
(71) Applicant: EditForce, Inc., Fukuoka-shi, Fukuoka 810-0001 (JP)
(72) Inventor: NAKAMORI, Masayuki, Osaka 565-0871 (JP); YAGI, Yusuke, Fukuoka 810-0001 (JP); IMAI, Takayoshi, Fukuoka 810-0001 (JP); OKII, Erika, Fukuoka 810-0001 (JP); TAMAI, Takayuki, Fukuoka 810-0001 (JP); NINOMIYA, Risa, Fukuoka 810-0001 (JP)
(74) Representative: Weickmann & Weickmann PartmbB
(86) International application number: PCT/JP2022/019038
(87) International publication number: WO 2022/230924

(57) **Abstract**

The object of the present invention is to provide a pharmaceutical composition or a method that is effective for treating myotonic dystrophy type 1 (DM1). Provided is a therapeutic drug or method for DM1 that utilizes a modified PPR protein.

## Description

### Technical Field

The present invention relates to a pharmaceutical composition or a method for treating myotonic dystrophy type 1.

### Background Art

Myotonic Dystrophy type 1 (hereinafter sometimes abbreviated as "DM1") is the most common muscle disease in adults. DM1 is an autosome-dominant multi-organ disorder that influences the skeletal muscle, the smooth muscle, as well as the eyeball, the heart, the endocrine system, and the central nervous system, and its symptoms are diverse, such as muscular atrophy, muscular weakness, myotonia, cataract, insulin resistance, hypogonadism, cardiac conduction disorder, anterior baldness, and intellectual impairment. The cause of DM1 is abnormal elongation of the CTG repeat sequence in the 3' UTR (untranslated region on 3' side) of the myotonin protein kinase (DMPK) gene. Splicing abnormality is evoked when a particular splicing control factor binds to a transcription product (mRNA) having an abnormally elongated CUG repeat sequence, resulting in deficiency of splicing control factors required for normal splicing.

Although development of therapeutic drugs for DM1 is currently brought forward with various approaches such as low molecular weight compounds and nucleic acid medicines (such as Non-Patent Literatures 1 and 2), there is no fundamental therapeutic drug at this stage, and treatment of DM1 is confined mainly to palliative therapy.

### Citation List

[Non-Patent Literature 1] Nakamori et al. Ann Clin Transl Neurol. (2015)
[Non-Patent Literature 2] Overby et al. Drug Discov. Today (2018)

### Summary of the Invention

### Problems to be Solved by the Invention

The object of the present invention is to provide a pharmaceutical composition or a method that is effective for treating myotonic dystrophy type 1 (DM1).

### Means for Solving the Problems

Pentatricopeptide repeat (PPR) protein is a protein that comprises a repeat of PPR motif that is about 35 amino acids long, and it is known that one PPR motif specifically or selectively binds to one base. In recent years, by virtue of the amino acid that functions when the PPR motif exerts the RNA binding property being identified, and the relationship between the PPR motif structure and the target base being clarified, development of RNA editing/modifying technology that utilizes the PPR protein is under way (such as WO 2013/058404).

As a result of searching for novel compounds/substances that may treat DM1, the present inventors found that DM1 may be treated by utilizing a modified PPR protein.

The present invention is based on the above knowledge, and may have the following characteristics.

[1]: A pharmaceutical composition for treating myotonic dystrophy type 1, wherein
   said pharmaceutical composition comprises a nucleic acid encoding a protein that specifically binds to the CUG repeat sequence,
   said protein that specifically binds to the CUG repeat sequence comprises at least 6 pentatricopeptide repeat (PPR) motifs consisting of polypeptides of 30 - 38 amino acids long represented by Formula 1,
      [Formula 1]

      **(HelixA)-X-(Helix B)-L** **(Formula 1)**

      [wherein in Formula 1:
      Helix A is a 12 amino acids long moiety that is capable of forming an α-helix structure, and is represented by Formula 2, [Formula 2]

      **A₁-A₂-A₃-A₄-A₅₋A₆-A₇-A₈-A₉-A₁₀-A₁₁-A₁₂** **(Formula 2)**

      wherein in Formula 2, A₁ - A₁₂ each independently represents an amino acid;
      X is absent, or is a moiety consisting of a length of 1 - 9 amino acids;
      Helix B is a moiety that is capable of forming an α-helix structure consisting of a length of 11 - 13 amino acids;
      L is a moiety represented by Formula 3 that is 2 - 7 amino acids long;
         [Formula 3]

         **Lᵥₗᵢ-Lᵥᵢ-Lᵥ-Lᵢᵥ-Lᵢᵢᵢ-Lᵢᵢ-Lᵢ** **(Formula 3)**
      and wherein in Formula 3, each amino acid is numbered from the C-terminal such as "i" (-1), "ii" (-2),
      provided that Lᵢᵢᵢ - Lᵥᵢᵢ may be absent]
      and the combination of three amino acids at A₁, A₄, and Lᵢᵢ, or the combination of two amino acids at A₄ and Lᵢᵢ in said each PPR motif is selected so that said each PPR motif binds to C, U, or G, and thereby configured so that said protein specifically binds to said CUG repeat sequence.

[2]: The pharmaceutical composition according to [1], wherein said protein that specifically binds to the CUG repeat sequence comprises 9 - 30 of said PPR motifs.
[3]: The pharmaceutical composition according to [2], wherein said protein that specifically binds to the CUG repeat sequence comprises 12 - 24 of said PPR motifs.
[4]: The pharmaceutical composition according to any of [1] to [3], wherein the combination of three amino acids at A₁, A₄, and Lᵢᵢ in said each PPR motif is:
   when the base to be the target for the PPR motif is A (adenine), the combination of three amino acids at A₁, A₄, and Lᵢᵢ is, in the order of (A₁, A₄, Lᵢᵢ), (valine, threonine, asparagine), (phenylalanine, serine, asparagine), (phenylalanine, threonine, asparagine), (isoleucine, asparagine, aspartic acid), or (threonine, threonine, asparagine);
   when the base to be the target for the PPR motif is G (guanine), the combination of three amino acids at A₁, A₄, and Lᵢᵢ is, in the order of (A₁, A₄, Lᵢᵢ), (glutamic acid, glycine, aspartic acid), (valine, threonine, aspartic acid), (lysine, threonine, aspartic acid), or (leucine, threonine, aspartic acid);
   when the base to be the target for the PPR motif is U (uracil), the combination of three amino acids at A₁, A₄, and Lᵢᵢ is, in the order of (A₁, A₄, Lᵢᵢ), (valine, asparagine, aspartic acid), (isoleucine, asparagine, asparagine), (isoleucine, asparagine, aspartic acid), (isoleucine, methionine, aspartic acid), (phenylalanine, proline, aspartic acid), or (tyrosine, proline, aspartic acid); or
   when the base to be the target for the PPR motif is C (cytosine), the combination of three amino acids at A₁, A₄, and Lᵢᵢ is, in the order of (A₁, A₄, Lᵢᵢ), (valine, asparagine, asparagine), (isoleucine, asparagine, asparagine), (valine, asparagine, serine), or (isoleucine, methionine, aspartic acid).
[5]: The pharmaceutical composition according to any of [1] to [3], wherein the combination of two amino acids at A₄ and Lᵢᵢ in said each PPR motif is:
   when the base to be the target for the PPR motif is A (adenine), the combination of two amino acids at A₄ and Lᵢᵢ is, in the order of (A₄, Lᵢᵢ), (threonine, asparagine), (serine, asparagine), or (glycine, asparagine);
   when the base to be the target for the PPR motif is G (guanine), the combination of two amino acids at A₄ and Lᵢᵢ is, in the order of (A₄, Lᵢᵢ), (threonine, aspartic acid), or (glycine, aspartic acid);
   when the base to be the target for the PPR motif is U (uracil), the combination of two amino acids at A₄ and Lᵢᵢ is, in the order of (A₄, Lᵢᵢ), (asparagine, aspartic acid), (proline, aspartic acid), (methionine, aspartic acid), or (valine, threonine);
   when the base to be the target for the PPR motif is C (cytosine), the combination of two amino acids at A₄ and Lᵢᵢ is, in the order of (A₄, Lᵢᵢ), (asparagine, asparagine), (asparagine, serine), or (leucine, aspartic acid).
[6]: The pharmaceutical composition according to any of [1] to [5], characterized in that said nucleic acid encoding a protein that specifically binds to the CUG repeat sequence is integrated into an expression vector.
[7]: The pharmaceutical composition according to any of [1] to [5], characterized in that said nucleic acid encoding a protein that specifically binds to the CUG repeat sequence is integrated into a viral vector.
[8]: The pharmaceutical composition according to [7], characterized in that said viral vector is a viral vector with tropism towards muscle tissue.
[9]: The pharmaceutical composition according to [8], characterized in that said viral vector is an adeno-associated virus (AAV) vector, an adenovirus vector, a retrovirus vector, a lentivirus vector, or a herpes simplex virus vector.
[10]: The pharmaceutical composition according to [9], characterized in that said viral vector is an AAV vector.
[11]: The pharmaceutical composition according to [10], characterized in that said AAV vector is an AAV1 vector, an AAV2 vector, an AAV6 vector, an AAV7 vector, an AAV8 vector, an AAV9 vector, an AAV10 vector, an AAV11 vector, or an AAV12 vector.
[12]: An expression vector comprising the nucleic acid encoding the protein that specifically binds to the CUG repeat sequence as defined in [1].
[13]: A cell comprising the expression vector according to [12].
[14]: A protein that specifically binds to the CUG repeat sequence that is produced from the cell according to [13].
[15]: A viral expression vector comprising the nucleic acid encoding the protein that specifically binds to the CUG repeat sequence as defined in [1].
[16]: A cell comprising the viral expression vector according to [15].
[17]: A viral vector that is produced from the cell according to [16].
[18]: The use of the nucleic acid encoding the protein that specifically binds to the CUG repeat sequence as defined in [1] in the manufacture of myotonic dystrophy type 1 therapeutic medicine.
[19]: A method for treating myotonic dystrophy type 1, comprising a step of applying to a subject a therapeutically effective amount of the pharmaceutical composition according to [1].

Note that an invention of any combination of one or more of the above characteristics is also encompassed by the scope of the present invention.

### Effects of the Invention

The symptoms of myotonic dystrophy type 1 (DM1) may be treated or alleviated by the present invention.

### Brief Description of the Drawings

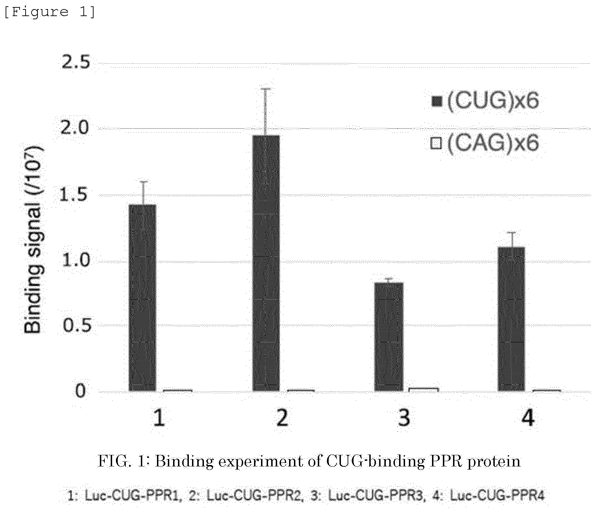

Figure 1 shows that the PPR protein of the present invention specifically bound to the CUG repeat sequence.

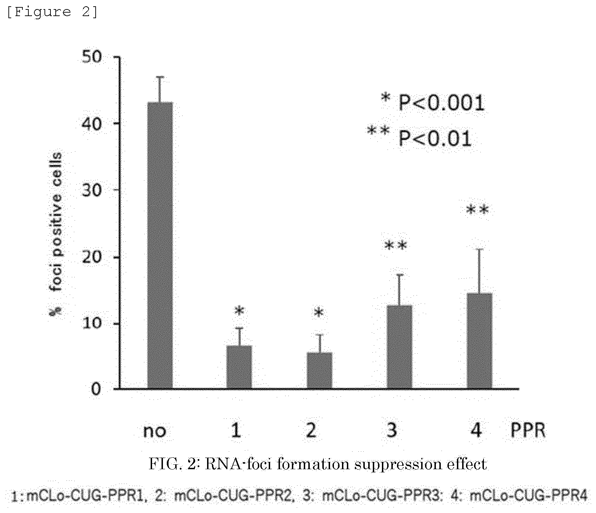

Figure 2 shows that the PPR protein of the present invention suppressed the formation of RNA-foci in DM1 model cells.

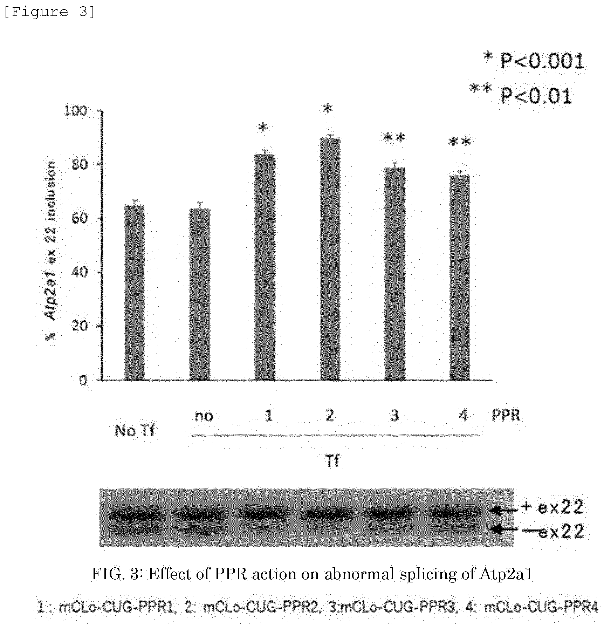

Figure 3 shows that the PPR protein of the present invention improved splicing abnormality in DM1 model cells.

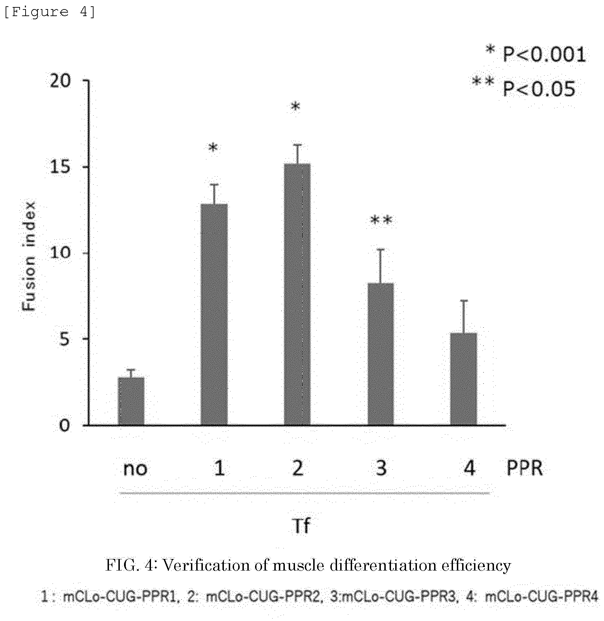

Figure 4 shows that the PPR protein of the present invention improved muscle differentiation efficiency in DM1 model animals.

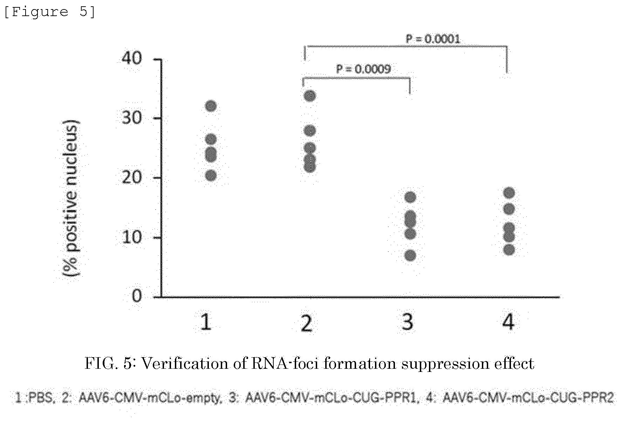

Figure 5 shows that the PPR protein of the present invention suppressed the formation of RNA-foci in DM1 model animals.

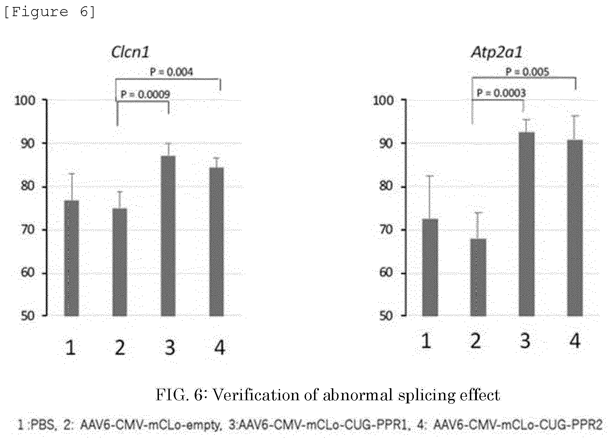

Figure 6 shows that the PPR protein of the present invention improved splicing abnormality in DM1 model animals.

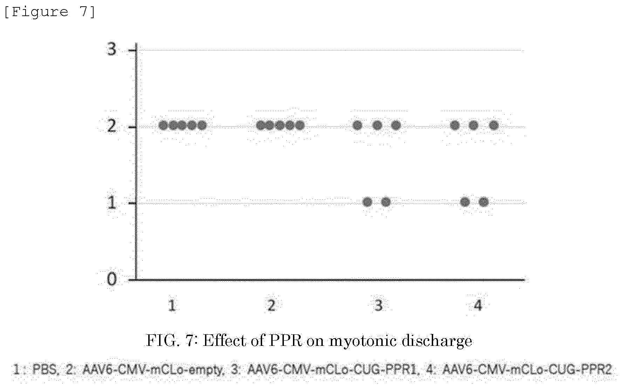

Figure 7 shows that the PPR protein of the present invention improved myotonia in DM1 model animals.

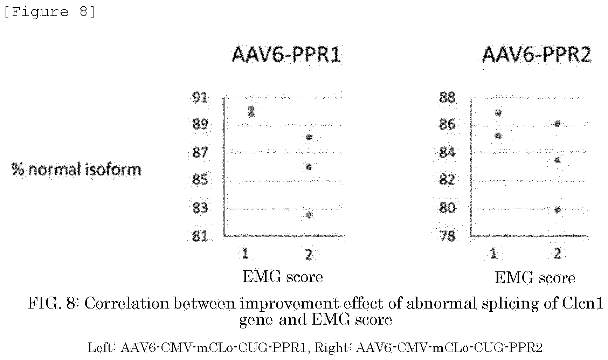

Figure 8 shows the relationship between the abnormal splicing improvement effect and the myotonia improvement effect by the present invention.

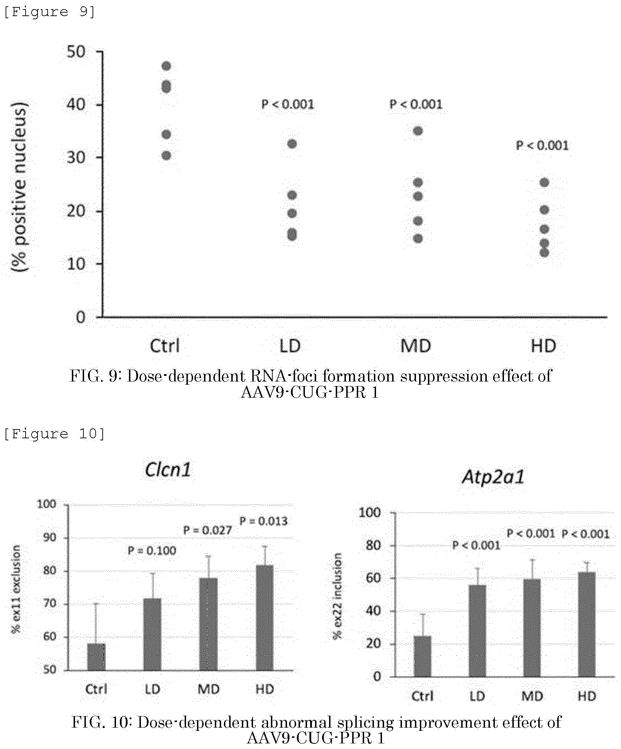

Figure 9 shows that AAV9-CUG-PPR 1 dose-dependently suppressed the formation of RNA-foci in DM1 model animals. In the Figure, "Ctrl" indicates the PBS administration group, "LD" indicates the AAV9-CUG-PPR 1 low dose administration group, "MD" indicates the AAV9-CUG-PPR 1 medium dose administration group, and "HD" indicates the AAV9-CUG-PPR 1 high dose administration group. The vertical axis shows the RNA-foci positive cell rate.
Figure 10 shows that AAV9-CUG-PPR 1 dose-dependently improved splicing abnormality in DM1 model animals. The left figure shows the results of the abnormal splicing detection system of Clcn1, the right figure of Atp2a1. In the figures, "Ctrl" indicates the PBS administration group, "LD" indicates the AAV9-CUG-PPR 1 low dose administration group, "MD" indicates the AAV9-CUG-PPR 1 medium dose administration group, and "HD" indicates the AAV9-CUG-PPR 1 high dose administration group. The vertical axis shows the respective content (%) of the normal splicing isoform.

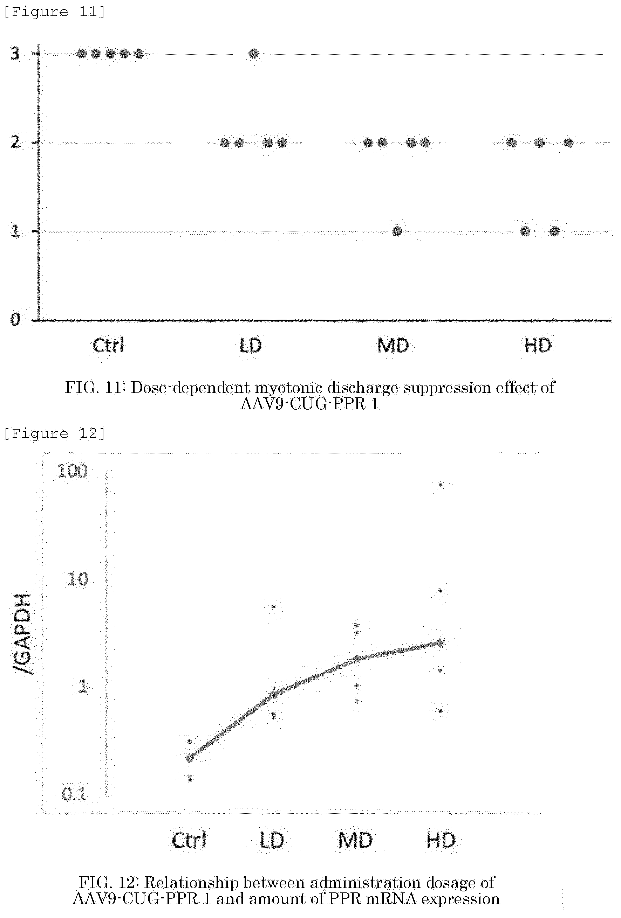

Figure 11 shows that AAV9-CUG-PPR 1 dose-dependently improved myotonia in DM1 model animals. The vertical axis shows the myotonia score, and the frequency of myotonic discharge decreases in the order of Scores 3, 2, 1, and 0. The frequency of the number of times of myotonic discharge and the severity are correlated. In the Figure, "Ctrl" indicates the PBS administration group, "LD" indicates the AAV9-CUG-PPR 1 low dose administration group, "MD" indicates the AAV9-CUG-PPR 1 medium dose administration group, and "HD" indicates the AAV9-CUG-PPR 1 high dose administration group.
Figure 12 shows the relationship between the administration dosage of AAV9-CUG-PPR 1 and the amount of PPR mRNA expression. GAPDH is used as the internal standard. Each of the individual points show the amount of PPR mRNA expression in the femoral muscle of each mouse individual. The vertical axis is shown in Log representation. In the Figure, "Ctrl" indicates the PBS administration group, "LD" indicates the AAV9-CUG-PPR 1 low dose administration group, "MD" indicates the AAV9-CUG-PPR 1 medium dose administration group, and "HD" indicates the AAV9-CUG-PPR 1 high dose administration group.

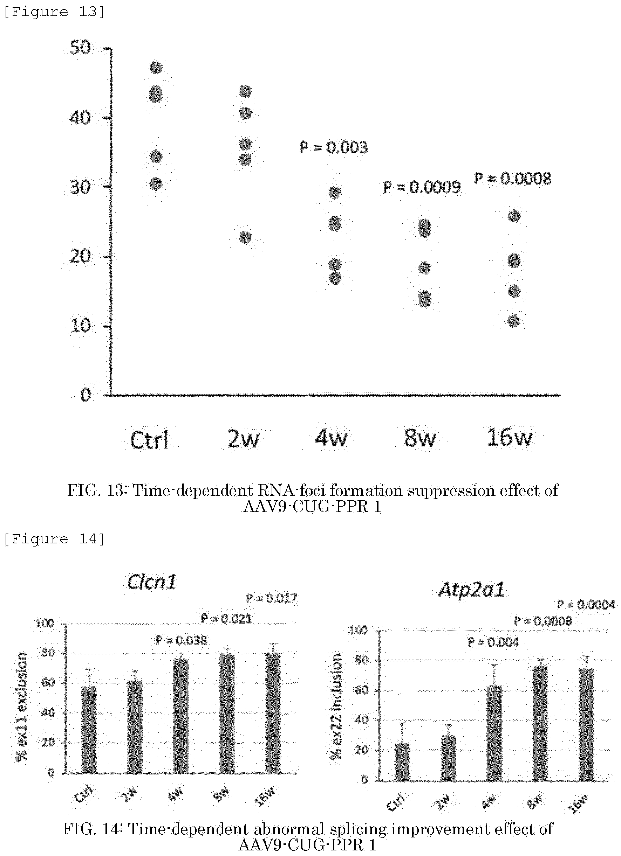

Figure 13 shows that AAV9-CUG-PPR 1 time-dependently suppressed the formation of RNA-foci in DM1 model animals. In the Figure, "Ctrl" indicates the PBS administration group. "2 w" "4 w" "8 w," and "16 w" each shows the test duration after administration of AAV9-CUG-PPR 1. The vertical axis shows the RNA-foci positive cell rate.
Figure 14 shows that AAV9-CUG-PPR 1 time-dependently improved splicing abnormality in DM1 model animals. The left figure shows the results of the abnormal splicing detection system of Clcn1, the right figure of Atp2a1. In the figures, "Ctrl" indicates the PBS administration group. "2 w" "4 w" "8 w," and "16 w" each shows the test duration after administration of AAV9-CUG-PPR 1. The vertical axis shows the respective content of the normal splicing isoform.

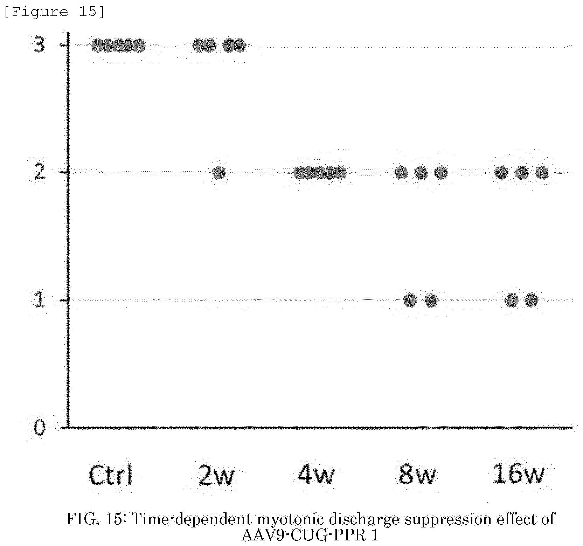

Figure 15 shows that AAV9-CUG-PPR 1 time-dependently improved myotonia in DM1 model animals. The vertical axis shows the myotonia score, and the frequency of myotonic discharge decreases in the order of Scores 3, 2, 1, and 0. The frequency of the number of times of myotonic discharge and the severity are correlated. In the Figure, "Ctrl" indicates the PBS administration group. "2 w" "4 w" "8 w," and "16 w" each shows the test duration after administration of AAV9-CUG-PPR 1.

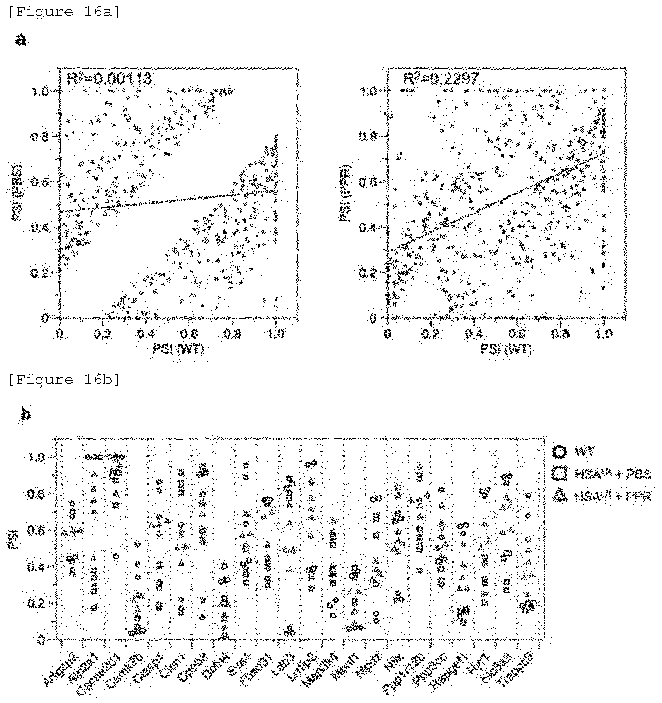

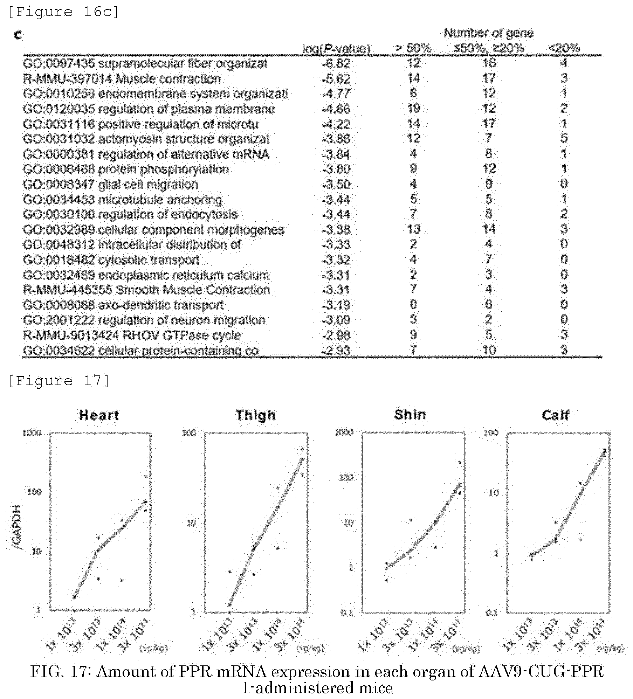

Figure 16 shows that splicing abnormality is comprehensively normalized after administration of AAV9-CUG-PPR 1. Figure 16a is a figure that shows how splicing events that had a change of 0.2 or more PSI (Percent spliced-in) between WT and PBS-administered HSA-LR mice (left) changed in the PPR-administered HSA-LR mice (right). Figure 16b is a figure that shows change in regard to DM1-related splicing events identified in Tanner et al. (2021). Black circles indicate signals of normal mice, blue rectangles indicate signals of the PBS-administered drug effect mice group, and red triangles indicate signals of the PPR-administered drug effect mice group. Figure 16c shows the result of gene ontology analysis on the genes of each point in Figure a. The P-value indicates the P value of each gene ontology term. The number of genes with exon skipping improvement rate are represented in the 3 stages of greater than 50%, 20 - 50%, and 20% or less for PPR-administered HSA-LR mice.
Figure 17 shows the amount of PPR mRNA expression in each organ of AAV9-CUG-PPR 1-administered mice. The vertical axis shows the relative expression amount when the expression amount of 1 x 10¹³ vg/kg-administered mice was set as 1 in Log representation. GAPDH mRNA expression amount is the internal standard. The horizontal axis shows the AAV9-CUG-PPR 1 dosage (vg/kg of mouse body weight). The values of each mouse individual are plotted with circles, and the median is connected with a line.

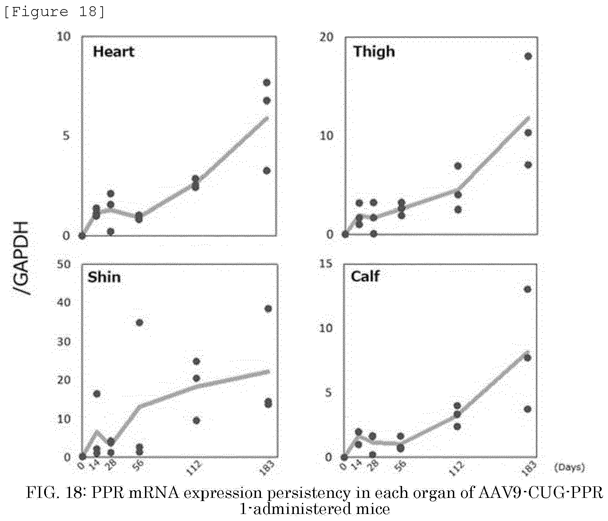

Figure 18 shows the persistency of mRNA expression in each organ of AAV9-CUG-PPR 1-administered mice. The vertical axis presents the relative expression amount when the expression amount of one AAV administration Week 2 (Day 14) mouse individual was set as 1. GAPDH mRNA expression amount is the internal standard. For the horizontal axis, "Ctrl" indicates the PBS group, and "14" "28" "56" "112," and "183" shows the number of days after administration. The values of each mouse individual are plotted with circles, and the average value is connected with a line.

### Description of Embodiments

### [PPR motif and PPR protein]

"PPR motif " in the present disclosure, unless particularly described, refers to a polypeptide, when analyzing the amino acid sequence with a protein domain search program on the web, composed of 30 - 38 amino acids having an amino acid sequence having an E value less than or equal to a predetermined value (desirably E-03) obtained as PF01535 in Pfam and PS51375 in Prosite. While the position numbers of the amino acids configuring the PPR motif as defined in the present disclosure are almost equivalent to PF01535, they correspond to numbers which are two subtracted from the amino acid positions of PS51375 (e.g. position 1 of the present invention -> position 3 of PS51375), provided that when referring to the amino acid at "ii" (-2) position, it is the second amino acid from the tail end (the C-terminal side) of the amino acids configuring the PPR motif, or two amino acids to the N-terminal side of the first amino acid of the next PPR motif, i.e. the amino acid at position -2. When the next PPR motif is not clearly identified, the amino acid that is two amino acids before the first amino acid of the next helix structure is set at "ii."

Although the conserved amino acid sequence of the PPR motif has low conservativeness at the amino acid level, the two α-helices are well conserved in the secondary structure. A typical PPR motif is composed of 35 amino acids, but its length is variable at 30 - 38 amino acids.

The basic skeleton of the PPR motif is known to be represented by Formula 1.

[Formula 4]

**(Helix A)-X-(Helix B)-L,** **(Formula 1)**

[wherein:
Helix A is a 12 amino acids long moiety that is capable of forming an α-helix structure, and is represented by Formula 2, [0035]
[Formula 5]

**A₁-A₂-A₃-A₄-A₅-A₆-A₇-A₇-A₉-A₁₀-A₁₁-A₁₂** **(Formula 2)**

wherein in Formula 2, A₁ - A₁₂ each independently represents an amino acid;
X is absent, or is a moiety consisting of a length of 1 - 9 amino acids;
Helix B is a moiety that is capable of forming an α-helix structure consisting of a length of 11 - 13 amino acids;
L is a moiety represented by Formula 3 that is 2 - 7 amino acids long;
   [Formula 6]

   **Lᵥᵢᵢ-Lᵥᵢ-Lᵥ-Lᵢᵥ-Lᵢᵢᵢ-Lᵢᵢ-Lᵢ** **(Formula 3)**
and wherein in Formula 3, each amino acid is numbered from the C-terminal such as "i" (-1), "ii" (-2),
provided that Lᵢᵢᵢ - Lᵥᵢᵢ may be absent].

"PPR protein " in the present disclosure, unless particularly described, refers to a PPR protein having one or more, preferably two or more of the aforementioned PPR motifs. "Protein " in the present disclosure, unless particularly described, refers to a general substance consisting of a polypeptide (a chain having multiple amino acids bound by peptide bonds), and also includes those consisting of polypeptides of relatively low molecular weight. "Amino acid" in the present invention may refer to an ordinary amino acid molecule, as well as to an amino acid residue that configures the peptide chain. To which it is referring to will be clear to those skilled in the art from the context.

"Selective" or "specific" in the present disclosure in regard to the binding ability of the PPR motif to an RNA base, unless particularly described, refers to the fact that the binding activity against any one base of the RNA bases is higher than the binding activity against other bases. This selectivity or specificity can be confirmed by those skilled in the art by planning experiments based on well-known methods, as well as be obtained from calculation by those skilled in the art.

RNA base in the present disclosure, unless particularly described, refers to the ribonucleotide base configuring the RNA, and specifically refers to any of adenine (A), guanine (G), cytosine (C), or uracil (U). Note that although the PPR protein may have selectivity against a base in the RNA, it will not bind to the nucleic acid monomer.

PPR proteins are abundantly present in plants, and 500 proteins, about 5000 motifs are found in Arabidopsis thaliana. PPR motifs and PPR proteins of diverse amino acid sequences are also present in many land plants such as rice, popular, and selaginella. In the present invention, naturally occurring PPR motifs and PPR proteins may be employed, or PPR motifs and PPR proteins designed based on the method disclosed in for example WO 2013/058404 may be utilized. Specifically, desired PPR motifs and PPR proteins can be designed based on the following information disclosed in WO 2013/058404 .

### (I) Information related to amino acid positions important for selective binding

The combination of three amino acids at 1, 4, and "ii" (-2) positions (A₁, A₄, Lᵢᵢ), or the combination of two amino acids at 4 and "ii" (-2) positions (A₄ and Lᵢᵢ) of the PPR motif is important for selective binding with the RNA base, and which RNA base it binds to can be determined by these combinations.

The present invention can utilize the knowledge related to the combination of three amino acids at A₁, A₄, and Lᵢᵢ and/or the combination of two amino acids at A₄ and Lᵢᵢ as disclosed in WO 2013/058404.

### (II) Information related to the correspondence between the combination of three amino acids at A₁, A₄, and Lii and the RNA base

(3-1) When the combination of three amino acids at A₁, A₄, and Lᵢᵢ is valine, asparagine, and aspartic acid in that order, the PPR motif thereof has selective RNA base binding ability that is binding strongly to U, in the next place binding to C, further followed by to A or G.
(3-2) When the combination of three amino acids at A₁, A₄, and Lᵢᵢ is valine, threonine, and asparagine in that order, the PPR motif thereof has selective RNA base binding ability that is binding strongly to A, in the next place binding to G, further followed by to C, but not binding to U.
(3-3) When the combination of three amino acids at A₁, A₄, and Lᵢᵢ is valine, asparagine, and asparagine in that order, the PPR motif thereof has selective RNA base binding ability that is binding strongly to C, in the next place binding to A or U, but not binding to G.
(3-4) When the combination of three amino acids at A₁, A₄, and Lᵢᵢ is glutamic acid, glycine, and aspartic acid in that order, the PPR motif thereof has selective RNA base binding ability that is binding strongly to G, but not binding to A, U, and C.
(3-5) When the combination of three amino acids at A₁, A₄, and Lᵢᵢ is isoleucine, asparagine, and asparagine in that order, the PPR motif thereof has selective RNA base binding ability that is binding strongly to C, in the next place to U, further followed by binding to A, but not binding to G.
(3-6) When the combination of three amino acids at A₁, A₄, and Lᵢᵢ is valine, threonine, and aspartic acid in that order, the PPR motif thereof has selective RNA base binding ability that is binding strongly to G, in the next place binding to U, but not binding to A and C.
(3-7) When the combination of three amino acids at A₁, A₄, and Lᵢᵢ is lysine, threonine, and aspartic acid in that order, the PPR motif thereof has selective RNA base binding ability that is binding strongly to G, in the next place binding to A, but not binding to U and C.
(3-8) When the combination of three amino acids at A₁, A₄, and Lᵢᵢ is phenylalanine, serine, and asparagine in that order, the PPR motif thereof has selective RNA base binding ability that is binding strongly to A, in the next place to C, further followed by binding to G and U.
(3-9) When the combination of three amino acids at A₁, A₄, and Lᵢᵢ is valine, asparagine, and serine in that order, the PPR motif thereof has selective RNA base binding ability that is binding strongly to C, in the next place binding to U, but not binding to A and G.
(3-10) When the combination of three amino acids at A₁, A₄, and Lᵢᵢ is phenylalanine, threonine, and asparagine in that order, the PPR motif thereof has selective RNA base binding ability that is binding strongly to A, but not binding to G, U, and C.
(3-11) When the combination of three amino acids at A₁, A₄, and Lᵢᵢ is isoleucine, asparagine, and aspartic acid in that order, the PPR motif thereof has selective RNA base binding ability that is binding strongly to U, in the next place binding to A, but not binding to G and C.
(3-12) When the combination of three amino acids at A₁, A₄, and Lᵢᵢ is threonine, threonine, and asparagine in that order, the PPR motif thereof has selective RNA base binding ability that is binding strongly to A, but not binding to G, U, and C.
(3-13) When the combination of three amino acids at A₁, A₄, and Lᵢᵢ is isoleucine, methionine, and aspartic acid in that order, the PPR motif thereof has selective RNA base binding ability that is binding strongly to U, in the next place binding to C, but not binding to A and G.
(3-14) When the combination of three amino acids at A₁, A₄, and Lᵢᵢ is phenylalanine, proline, and aspartic acid in that order, PPR, motifs thereof has selective RNA base binding ability that is binding strongly to U, in the next place binding to C, but not binding to A and G.
(3-15) When the combination of three amino acids at A₁, A₄, and Lᵢᵢ is tyrosine, proline, and aspartic acid in that order, the PPR motif thereof has selective RNA base binding ability that is binding strongly to U, but not binding to A, G, and C.
(3-16) When the combination of three amino acids at A₁, A₄, and Lᵢᵢ is leucine, threonine, and aspartic acid in that order, the PPR motif thereof has selective RNA base binding ability that is binding strongly to G, but not binding to A, U, and C.

### (II) Information related to the correspondence between the combination of two amino acids at A₄ and Lᵢᵢ and the RNA base

(2-1) When A₄ and Lᵢᵢ is asparagine and aspartic acid in that order, the PPR motif thereof has selective RNA base binding ability that is binding strongly to U, in the next place to C, further followed by binding to A and G.
(2-2) When A₄ and Lᵢᵢ is asparagine and asparagine in that order, the PPR motif thereof has selective RNA base binding ability that is binding strongly to C, in the next place to U, further followed by binding to A and G.
(2-3) When A₄ and Lᵢᵢ is threonine and asparagine in that order, the PPR motif thereof has selective RNA base binding ability that is binding strongly to A, followed by weakly binding to G, U, and C.
(2-4) When A₄ and Lᵢᵢ is threonine and aspartic acid in that order, the PPR motif thereof has selective RNA base binding ability that is binding strongly to G, followed by weakly binding to A, U, and C.
(2-5) When A₄ and Lᵢᵢ is serine and asparagine in that order, the PPR motif thereof has selective RNA base binding ability that is binding strongly to A, in the next place binding to G, U, and C.
(2-6) When A₄ and Lᵢᵢ is glycine and aspartic acid in that order, the PPR motif thereof has selective RNA base binding ability that is binding strongly to G, in the next place to U, further followed by binding to A, but not binding to C.
(2-7) When A₄ and Lᵢᵢ is asparagine and serine in that order, the PPR motif thereof has selective RNA base binding ability that is binding strongly to C, in the next place to U, further followed by binding to A and G.
(2-8) When A₄ and Lᵢᵢ is proline and aspartic acid in that order, the PPR motif thereof has selective RNA base binding ability that is binding strongly to U, in the next place binding to G, C, and C, but not binding to A.
(2-9) When A₄ and Lᵢᵢ is glycine and asparagine in that order, the PPR motif thereof has selective RNA base binding ability that is binding strongly to A, in the next place binding to G, but not binding to C and U.
(2-10) When A₄ and Lᵢᵢ is methionine and aspartic acid in that order, the PPR motif thereof has selective RNA base binding ability that is binding strongly to U, followed by weakly binding to A, G, and C.
(2-11) When A₄ and Lᵢᵢ is leucine and aspartic acid in that order, the PPR motif thereof has selective RNA base binding ability that is binding strongly to C, in the next place binding to U, but not binding to A and G.
(2-12) When A₄ and Lᵢᵢ is valine and threonine in that order, the PPR motif thereof has selective RNA base binding ability that is binding strongly to U, in the next place binding to A, but not binding to G and C.

Note that the above rules mean that the possibility that the PPR motif binds to the target base may be increased with statistical significance by the amino acid combination at (A₁, A₄, Lᵢᵢ) or (A₄ and Lᵢᵢ) of the PPR motif, and it does not mean that a PPR motif (or PPR protein) that binds to the target base (or base sequence) with 100% probability may be manufactured by the above rules. However, those skilled in the art will be able to obtain a PPR motif (or PPR protein) that binds to the target base (or base sequence) with high probability by manufacturing about several to several tens of types of candidate PPR proteins based on the above rules. Note that the selection of the desired PPR protein through manufacture and confirmation of candidate PPR proteins may be performed within the scope of ordinary trial and error by those skilled in the art, and shall not be excessive burden to those skilled in the art.

### [Utilization of PPR motifs and PPR proteins]

One PPR motif may recognize a particular base of RNA. Further, based on the present invention, by appropriately selecting the amino acids at particular positions, selective PPR motifs can be selected or designed for each of A, U, G, and C. Furthermore, a protein comprising an appropriate series of such PPR motifs may specifically or selectively recognize the corresponding base sequence. Further, a PPR motif that is capable of selectively binding to a desired RNA base, and a protein having multiple PPR motifs capable of sequence-specifically binding to a desired RNA can be designed with the aforementioned knowledge. Upon designing, the sequence information of the native form PPR motif may be referred for portions other than the amino acids at the important positions in the PPR motif. Moreover, designing may be performed by employing the native form PPR motif in general and substituting only the said amino acids at the important positions. The number of repeats of the PPR motif can be set appropriately according to the target sequence, for example it can be two or more, and can be 2 - 30.

### [PPR protein that specifically binds to the CUG repeat sequence]

The present invention relates to a PPR protein that specifically binds to an mRNA comprising abnormally elongated CUG repeat sequence that is the cause of DM1. The PPR protein according to the present invention can be produced by linking multiple sets of [a PPR motif that binds to C, a PPR motif that binds to U, a PPR motif that binds to G] based on the aforementioned theory. Note that the first PPR motif of the PPR protein according to the present invention does not necessarily need to be a PPR motif that binds to C, and the first PPR motif may be a PPR motif that binds to U or a PPR motif that binds to G as long as the PPR protein specifically binds to the CUG repeat sequence as a whole. Moreover, naturally, as long as the PPR protein according to the present invention has the configuration to specifically bind to the CUG repeat sequence as a whole, the number of PPR motifs contained in the PPR protein does not need to be a multiple of 3.

Non-limiting examples of the C-binding PPR motif that may be employed in the present invention can include PPR motifs having the following sequences.

VTYNTLIDGLCKSGKIEEALKLFKEMEEKGITPSV (SEO ID NO. 1)
VTYNTNtDQLCKSGKIEEALKLEKEMEEKGITPSV (SEO ID NO. 2)
VTYNTNIDQLCKSGRIEEALKLFKEMEEKGITPSV (SEQ ID NO. 3)
VTYNTLIDGLCKSGRIEEALKLFKEMEEKGITPSV (SEO ID NO. 4)

In the present invention, provided that the amino acids at positions A₁, A₄, and Lᵢᵢ of the above sequence or the amino acids at positions A₄ and Lᵢᵢ are conserved, a PPR motif having a sequence homology (or sequence identity) of 80% or more (preferably, 85% or more, 90% or more, 95% or more, 96% or more, 97% or more, 98% or more, 99% or more) with the above sequence, which has the ability to bind to C may be employed.

In the present invention, provided that the amino acids at positions A₁, A₄, and Lᵢᵢ of the above sequence or the amino acids at positions A₄ and Lᵢᵢ are conserved, a PPR motif having substitutions, additions, and/or deletions of 7 bases or less (i.e., 1, 2, 3, 4, 5, 6, or 7 bases) to the above sequence, which has the ability to bind to C may be employed. Note that the amino acid substitution may be for example a conservative substitution of amino acids known in the art.

Non-limiting examples of the U-binding PPR motif that may be employed in the present invention can include PPR motifs having the following sequences.

VTVNTLIDGLCKAGRLDEAEELLEEMEEKGIKPDV (SEO ID NO. 5)
VTYNTLIDGLCKAGKLDEAEELLEEMEEMKGIKPDV (SEO ID NO. 6)

In the present invention, provided that the amino acids at positions A₁, A₄, and Lᵢᵢ of the above sequence or the amino acids at positions A₄ and Lᵢᵢ are conserved, a PPR motif having a sequence homology (or sequence identity) of 80% or more (preferably, 85% or more, 90% or more, 95% or more, 96% or more, 97% or more, 98% or more, 99% or more) with the above sequence, which has the ability to bind to U may be employed.

In the present invention, provided that the amino acids at positions A₁, A₄, and Lᵢᵢ of the above sequence or the amino acids at positions A₄ and Lᵢᵢ are conserved, a PPR motif having substitutions, additions, and/or deletions of 7 bases or less (i.e., 1, 2, 3, 4, 5, 6, or 7 bases) to the above sequence, which has the ability to bind to U may be employed. Note that the amino acid substitution may be for example a conservative substitution of amino acids known in the art.

Non-limiting examples of the G-binding PPR motif that may be employed in the present invention can include PPR motifs having the following sequences.

VTYTTLIDGLCKAGKVDEALELFDEMKERGIKPDV (SEQ ID NO. 7)
VTYTTUDGLCKAGKVDEALELFDEMKERGIKPDE (SEQ ID NO. 8)
VTYTTLIDGLCKAGRXTDFALELFDEMKERGIKPDV (SEQ ID NO. 9)
VTYTTLIDGLCKAGRVDEALELFDEMKERGIKPDE (SEQ ID NO. 10)

In the present invention, provided that the amino acids at positions A₁, A₄, and Lᵢᵢ of the above sequence or the amino acids at positions A₄ and Lᵢᵢ are conserved, a PPR motif having a sequence homology (or sequence identity) of 80% or more (preferably, 85% or more, 90% or more, 95% or more, 96% or more, 97% or more, 98% or more, 99% or more) with the above sequence, which has the ability to bind to G may be employed.

In the present invention, provided that the amino acids at positions A₁, A₄, and Lᵢᵢ of the above sequence or the amino acids at positions A₄ and Lᵢᵢ are conserved, a PPR motif having substitutions, additions, and/or deletions of 7 bases or less (i.e., 1, 2, 3, 4, 5, 6, or 7 bases) to the above sequence, which has the ability to bind to G may be employed. Note that the amino acid substitution may be for example a conservative substitution of amino acids known in the art.

When referring to "identity" herein in relation to a base sequence (sometimes referred to as nucleotide sequence) or an amino acid sequence, unless particularly described, it means the percentage of the number of matched bases or amino acids shared between the two sequences when two sequences are aligned in an optimal aspect. In other words, it can be calculated by identity = (number of matched positions/total number of positions) x 100, and can be calculated with algorithms which are distributed for a charge or for free. Further, such algorithms are integrated within e.g. NBLAST and XBLAST programs described in Altschul et al., J. Mol. Biol. 215 (1990) 403-410. More specifically, search/analysis related to the identity of the base sequence or the amino acid sequence can be performed by algorithms or programs well-known to those skilled in the art (such as BLASTN, BLASTP, BLASTX, ClustalW). The parameters when employing the programs can be appropriately set by those skilled in the art, and default parameters of each program may also be employed. The specific methods of these analysis methods are also well-known to those skilled in the art.

Conservative substitution of amino acids may be for example a substitution to amino acids with similar property. Amino acids with similar property refer to for example an amino acid with similar physical properties such as hydropathy, charge, pKa, and solubility, and for example refer to the following.
- Hydrophobic amino acids: alanine, valine, glycine, isoleucine, leucine, phenylalanine, proline, tryptophan, tyrosine;
- Non-hydrophobic amino acids: arginine, asparagine, aspartic acid, glutamic acid, glutamine, lysine, serine, threonine, cysteine, histidine;
- Hydrophilic amino acids: arginine, asparagine, aspartic acid, glutamic acid, glutamine, lysine, serine, threonine;
- Acidic amino acids: aspartic acid, glutamic acid;
- Basic amino acids: lysine, arginine, histidine;
- Neutral amino acids: alanine, asparagine, cysteine, glutamine, glycine, isoleucine, leucine, methionine, phenylalanine, proline, serine, threonine, tryptophan, tyrosine, valine;
- Sulfur-containing amino acids: methionine, cysteine;
- Aromatic ring-containing amino acids: tyrosine, tryptophan, phenylalanine .

### [Treatment of DM1 by the present invention]

The effects of the present invention are exerted when the PPR protein of the present invention binds to an mRNA comprising an abnormally elongated CUG repeat sequence within the subject cell. The delivery means for the PPR protein into the subject cell is not limited, and a nucleic acid encoding the PPR protein of the present invention may be delivered into the subject cell and allow the expression of the PPR protein of the present invention within the subject cell, or the PPR protein of the present invention per se may be delivered into the subject cell. The delivery means for the protein or nucleic acid is not limited, and various means known in the art can be employed. Non-limiting examples of the delivery means for the nucleic acid or protein into the subject cell that can be employed in the present invention can include liposomes, lipid nanoparticles (LNP), high molecular weight micelles, emulsions, high molecular weight microcapsules, antibody-nucleic acid complexes, antibody-drug complexes, viruses, and the like.

Non-limiting examples of viral vectors that may be employed in the present invention can include adeno-associated virus (AAV) vectors, adenovirus vectors, retrovirus vectors, lentivirus vectors, or herpes simplex virus vectors, and it can be appropriately selected by those skilled in the art depending on the tropism towards cells or tissues, the necessity of integration of nucleic acid into the host genome, and the like.

AAV vectors can be particularly favorably employed in the present invention because they have the ability to infect both dividing cells and resting cells, and can transfer genetic material to extremely diverse cell types. Twelve types of AAV serum types (AAV1 - AAV12) have been reported to date, and all of the known serum type can infect various types of tissue cells. The serum type of the AAV vectors that may be employed in the present invention is not limited, and for example an AAV1 vector, an AAV2 vector, an AAV6 vector, an AAV7 vector, an AAV8 vector, an AAV9 vector, an AAV10 vector, an AAV11 vector, or an AAV12 vector that are known to have tropism towards muscle tissue (skeletal muscle, cardiac muscle, and/or smooth muscle) may be employed.

Treatment of DM1 employing the present invention may be an aspect of directly applying the present invention to the living body of a subject suffering from DM1 (in vivo), or may be an aspect of treating the cells or tissues outside the living body with the present invention and then introducing into the living body of a subject (ex vivo).

The method for producing an expression vector comprising a nucleic acid encoding the PPR protein of the present invention is not limited, and production can be done by those skilled in the art by conventional means (such as use of commercially available protein expression vector production kits, use of commercially available viral expression vector production kits, and consignment to manufacturing companies).

The method for producing the PPR protein of the present invention is not limited, and for example, a desired PPR protein can be obtained by selectively extracting and/or purifying the PPR protein by conventional means from a cell (such as an animal cell, a plant cell, E. coli, and yeast) comprising an expression vector (such as a plasmid, a transposon, and a virus) encoding the PPR protein designed by the methods described herein.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### [Example 1: Production of PPR protein that binds to CUG repeat RNA sequence and binding analysis to RNA]

Recombinant proteins were produced for production of PPR protein that binds to the CUG repeat RNA sequence (hereinafter abbreviated as CUG-PPR) and binding analysis thereof, and RNA binding experiment was carried out.

### <Experimental materials and methods>

### (1) Preparation of PPR expression plasmid vector

A gene fused in the order of luciferase, PPR protein, and 3 x hexahistidine tag was cloned into multiple cloning sites of E. coli expression plasmid vector pET-22b. The expression of the fusion gene is controlled by a T7 promoter. Cloning of the gene having the correct size was confirmed by PCR method, and the gene sequence was confirmed by sequencing (SEQ ID NOs. 11 - 14). These were designated Luc-CUG-PPR 1, Luc-CUG-PPR 2, Luc-CUG-PPR 3, and Luc-CUG-PPR 4, and employed in subsequent tests.

### [Table 1]

**[Table 1]**

| Table 1. Amino acid sequences encoded by the cloned genes | |
|---|---|
| Protein name | Sequence (PPR protein is underlined) |
| Luc - CUG - PPR1 (SEQ ID. NO. 11) | MAGVFTLEDFVGDWRQTAGYNLDQVLEQGGVSSLFQNLGVSVTPIQRIVLS GENGLKIDIHVIIPYEGLSGDQMGQIEKIFKVVYPVDDHHFKVILHYGTLVID GVTPNMIDYFGRPYEGIAVFDGKKITVTGTLWNGNKIIDERLINPDGSLLFRV TINGVTGWRLCERILAGGGGSLVPRGSGGGGSSRLHMGNSVVTYNTLIDGLC KSGKIEEALKLFKEMEEKGITPSVVTYNTLIDGLCKAGRLDEAEELLEEMEEKGI KPDVVTYTTLIDGLCKAGKVDEALELFDEMKERGIKPDVVTYNTLIDGLCKSG KIEEALKLFKEMEEKGITPSVVTYNTLIDGLCKAGRLDEAEELLEEMEEKGIKPD VVTYTTLIDGLCKAGKVDEALELFDEMKERGIKPDVVTYNTLIDGLCKSGKIEE ALKLFKEMEEKGITPSVVTYNTLIDGLCKAGRLDEAEELLEEMEEKGIKPDVVT YTTLIDGLCKAGKVDEALELFDEMKERGIKPDVVTYNTLIDGLCKSGKIEEALK LFKEMEEKGITPSVVTYNTLIDGLCKAGRLDEAEELLEEMEEKGIKPDVVTYTT LIDGLCKAGKVDEALELFDEMKERGIKPDVVTYNTLIDGLCKSGKIEEALKLFK EMEEKGITPSVVTYNTLIDGLCKAGRLDEAEELLEEMEEKGIKPDVVTYTTLID GLCKAGKVDEALELFDEMKERGIKPDVVTYNTLIDGLCKSGKIEEALKLFKEME EKGITPSVVTYNTLIDGLCKAGRLDEAEELLEEMEEKGIKPDVVTYTTLIDGLC KAGKVDEALELFDEMKERGIKPDELTYNTLISGLGKAGRARDPPVLSSGSGGS GGGGHHHHHHGR |
| Luc - CUG - PPR2 (SEQ ID NO. 12) | MAGVFTLEDFVGDWRQTAGYNLDQVLEQGGVSSLFQNLGVSVTPIQRIVLS GENGLKIDIHVIIPYEGLSGDQMGQIEKIFKVVYPVDDHHFKVILHYGTLVID GVTPNMIDYFGRPYEGIAVFDGKKITVTGTLWNGNKIIDERLINPDGSLLFRV TINGVTGWRLCERILAGGGGSLVPRGSGGGGSSRLHMGNSVVTYNTNIDQL CKSGKIEEALKLFKEMEEKGITPSVVTYNTLIDGLCKAGRLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGRLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGRLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGRLDEAEELLEEMEEKGIKPDVVTY TTLIDGLCKAGKVDEALELFDEMKERGIKPDVVTYNTLIDGLCKSGKIEEALKL FKEMEEKGITPSVVTYNTLIDGLCKAGRLDEAEELLEEMEEKGIKPDVVTYTTL IDGLCKAGKVDEALELFDEMKERGIKPDVVTYNTLIDGLCKSGKIEEALKLFKE MEEKGITPSVVTYNTLIDGLCKAGRLDEAEELLEEMEEKGIKPDVVTYTTLIDG LCKAGKVDEALELFDEMKERGIKPDELTYNTLISGLGKAGRARDPPVLSSGSG GSGGGGHHHHHHGR |
| L tic -CUG - PPR3 (SEQ ID NO. 13) | MAGVFTLEDFVGDWRQTAGYNLDQVLEQGGVSSLFQNLGVSVTPIQRIVLS GENGLKIDIHVIIPYEGLSGDQMGQIEKIFKVVYPVDDHHFKVILHYGTLVID GVTPNMIDYFGRPYEGIAVFDGKKITVTGTLWNGNKIIDERLINPDGSLLFRV TINGVTGWRLCERILAGGGGSLVPRGSGGGGSSRLHMGNSVVTYNTNIDQL CKSGKIEEALKLFKEMEEKGITPSVVTYNTLIDGLCKAGKLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGKLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGKLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGKLDEAEELLEEMEEKGIKPDVVTY TTLIDGLCKAGKVDEALELFDEMKERGIKPDVVTYNTLIDGLCKSGKIEEALKL FKEMEEKGITPSVVTYNTLIDGLCKAGKLDEAEELLEEMEEKGIKPDVVTYTTL IDGLCKAGKVDEALELFDEMKERGIKPDVVTYNTLIDGLCKSGKIEEALKLFKE MEEKGITPSVVTYNTLIDGLCKAGKLDEAEELLEEMEEKGIKPDVVTYTTLIDG LCKAGKVDEALELFDEMKERGIKPDELTYNTLISGLGKAGRARDPPVLSSGSG GSGGGGHHHHHHGR |
| Luc - CUG - PPR4 (SEQ ID NO. 14) | MAGVFTLEDFVGDWRQTAGYNLDQVLEQGGVSSLFQNLGVSVTPIQRIVLS GENGLKIDIHVIIPYEGLSGDQMGQIEKIFKVVYPVDDHHFKVILHYGTLVID GVTPNMIDYFGRPYEGIAVFDGKKITVTGTLWNGNKIIDERLINPDGSLLFRV TINGVTGWRLCERILAGGGGSLVPRGSGGGGSSRLHMGNSVVTYNTNIDQL CKSGRIEEALKLFKEMEEKGITPSVVTYNTLIDGLCKAGRLDEAEELLEEMEEK GIKPDVVTYTTLIDGLCKAGRVDEALELFDEMKERGIKPDVVTYNTLIDGLCKS GRIEEALKLFKEMEEKGITPSVVTYNTLIDGLCKAGRLDEAEELLEEMEEKGIKP DVVTYTTLIDGLCKAGRVDEALELFDEMKERGIKPDVVTYNTLIDGLCKSGRIE EALKLFKEMEEKGITPSVVTYNTLIDGLCKAGRLDEAEELLEEMEEKGIKPDVV TYTTLIDGLCKAGRVDEALELFDEMKERGIKPDVVTYNTLIDGLCKSGRIEEAL KLFKEMEEKGITPSVVTYNTLIDGLCKAGRLDEAEELLEEMEEKGIKPDVVTYT TLIDGLCKAGRVDEALELFDEMKERGIKPDVVTYNTLIDGLCKSGRIEEALKLF KEMEEKGITPSVVTYNTLIDGLCKAGRLDEAEELLEEMEEKGIKPDVVTYTTLI DGLCKAGRVDEALELFDEMKERGIKPDVVTYNTLIDGLCKSGRIEEALKLFKE MEEKGITPSVVTYNTLIDGLCKAGRLDEAEELLEEMEEKGIKPDVVTYTTLIDG LCKAGRVDEALELFDEMKERGIKPDELTYNTLISGLGKAGRARDPPVLSSGSG GSGGGGHHHHHHGR |

The details of the sequences of the PPR motifs contained in the PPR protein in the above Table 1 are shown below. All of the PPR proteins comprise 18 PPR motifs.

**[Table 2]**

| Table 2. Amino acid sequences encoded by the cloned genes | |
|---|---|
| Protein name | Sequence (PPR protein is underlined) |
| mCLo - CU G - PPR1 (SEQ ID NO. 17) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTLID GLCKSGKIEEALKLFKEMEEKGITPSVVTYNTLIDGLCKAGRLDEAEELLEEME EKGIKPDVVTYTTLIDGLCKAGKVDEALELFDEMKERGIKPDVVTYNTLIDGL CKSGKIEEALKLFKEMEEKGITPSVVTYNTLIDGLCKAGRLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGRLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGRLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGRLDEAEELLEEMEEKGIKPDVVTY TTLIDGLCKAGKVDEALELFDEMKERGIKPDVVTYNTLIDGLCKSGKIEEALKL FKEMEEKGITPSVVTYNTLIDGLCKAGRLDEAEELLEEMEEKGIKPDVVTYTTL IDGLCKAGKVDEALELFDEMKERGIKPDELTYNTLISGLGKAGRARDPPVLSS PKKKRKVEDPKKKRKVEDPKKKRKVGSDYKDHDGDYKDHDIDYKDDDDK MAGR |
| mCLo - CU G - PPR2 (SEQ ID NO. 18) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTNID QLCKSGKIEEALKLFKEMEEKGITPSVVTYNTLIDGLCKAGRLDEAEELLEEME EKGIKPDVVTYTTLIDGLCKAGKVDEALELFDEMKERGIKPDVVTYNTLIDGL CKSGKIEEALKLFKEMEEKGITPSVVTYNTLIDGLCKAGRLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGRLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGRLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGRLDEAEELLEEMEEKGIKPDVVTY |

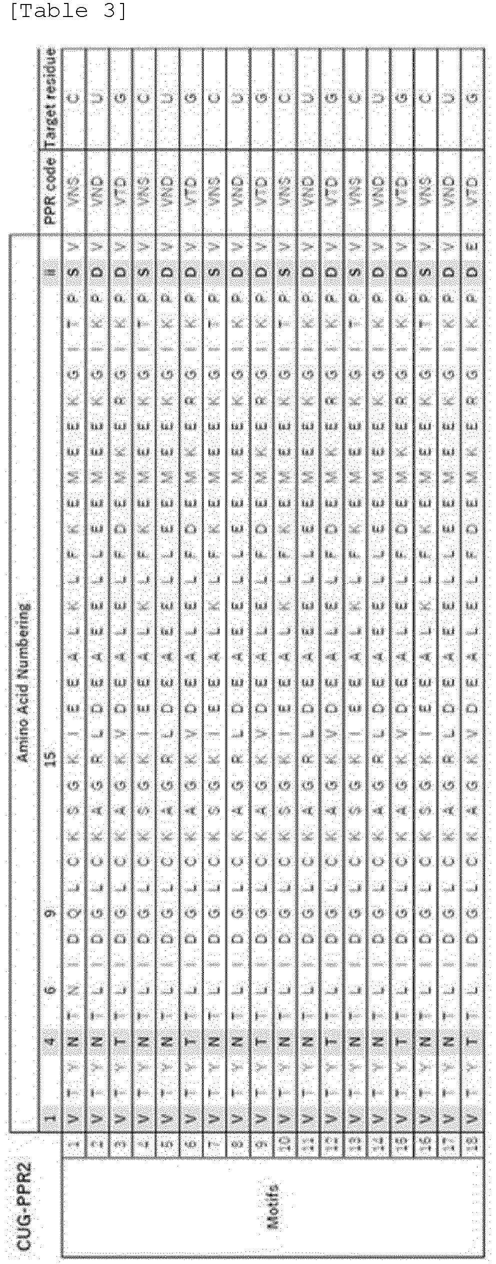

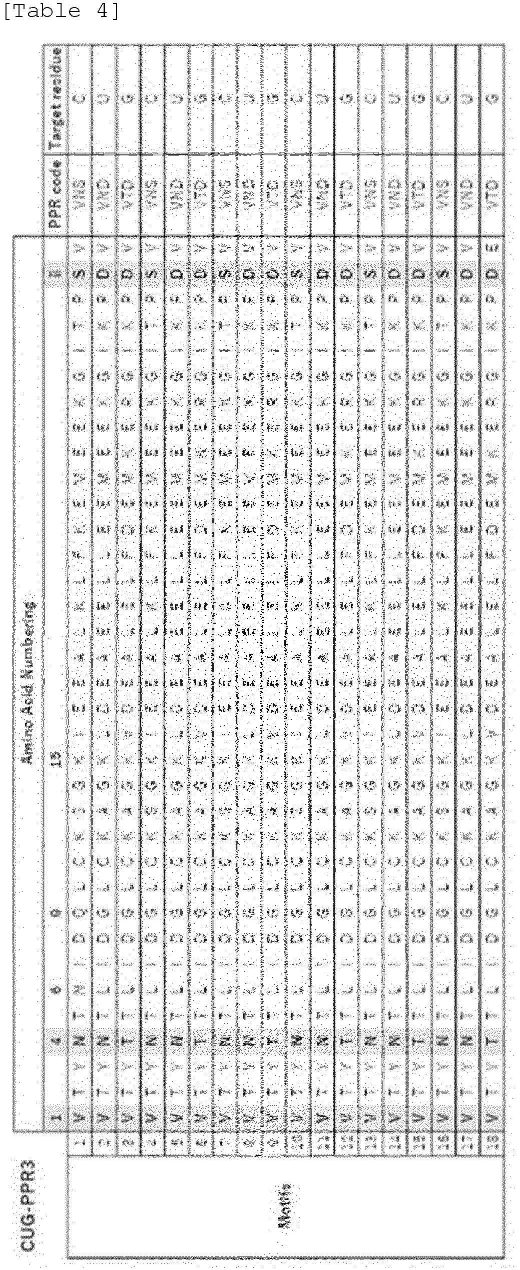

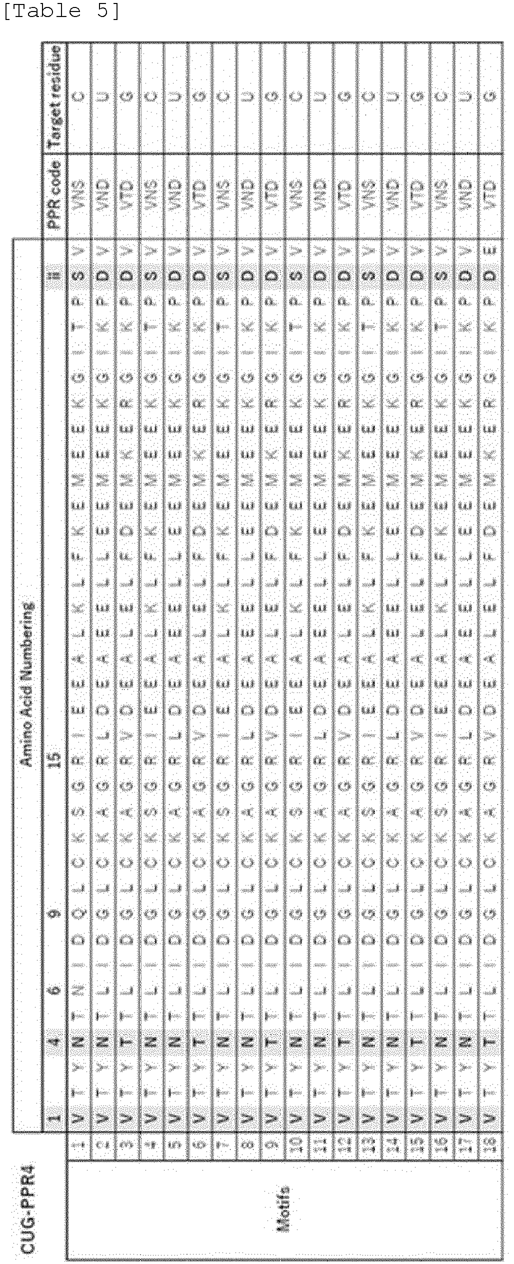

### (2) Preparation of PPR protein

The expression plasmid vector constructed as above was gene transferred into E. coli Rosetta (DE3) strain. This E. coli was cultured in 2 mL of LB medium containing 100 µg/µL ampicillin at 37°C for 12 hours. The culture medium was transferred to an incubator at 15°C when OD₆₀₀ reached 0.5 to 0.8, and left still for 30 minutes. Then, 100 µL (final concentration 0.1 mM IPTG) was added, and this was cultured at 15°C for 16 hours. Centrifugation was performed at 5,000 x g, 4°C, for 10 minutes, the E. coli pellets were collected, 1.5 mL of lysis buffer (20 mM Tris-HCl, pH 8.0, 150 mM NaCl, 0.5% NP-40, 1 mM MgCl₂, 2 mg/ml lysozyme, 1 mM PMSF, 2 µl of DNase) was added, and this was frozen at -80°C for 20 minutes. Frozen homogenization of cells was carried out at 25°C for 30 minutes with shaking. Subsequently, centrifugation operation was performed at 3700 rpm, 4°C, for 15 minutes, and the supernatant comprising soluble PPR protein (E. coli lysate) was collected and employed for the following experiment.

### (3) Binding test of PPR protein to RNA

The binding test between the PPR protein and RNA was carried out with a binding experiment method between the PPR protein and biotinylated RNA on a streptavidin plate. RNA probes were synthesized to have biotin modified at the 5'-terminal of each of a 30-base RNA comprising the target CUG x 7 sequence (GACACUGCUGCUGCUGCUGCUGCUGAUGCA (SEQ ID NO. 15)) and a 30-base RNA comprising a non-target CAG x 6 (GACAUGCCAGCAGCAGCAGCAGCAGGACUG (SEQ ID NO. 16)) (consigned to Greiner). 2.5 pmol of the biotinylated RNA probes were added to a streptavidin-coated plate (Cat No. 15502, Thermo fisher), and allowed to react for 30 minutes at room temperature. This was washed with probe washing buffer (20 mM Tris-HCl (pH 7.6), 150 mM NaCl, 5 mM MgCl₂, 0.5% NP-40, 1 mM DTT, 0.1% BSA). A well with lysis buffer without adding biotinylated RNA was also prepared for background measurement (designated "- Probe"). Then, blocking buffer (20 mM Tris-HCl (pH 7.6), 150 mM NaCl, 5 mM MgCl₂, 0.5% NP-40, 1 mM DTT, 1% BSA) was added, and the plate surface was blocked for 30 minutes at room temperature. One hundred microliters of the E. coli lysate comprising luciferase-fusion PPR protein having 1.5 x 10⁸ LU/µL of luminescence amount was added to the wells, and binding reaction was performed for 30 minutes at room temperature. Washing with 200 µL of washing buffer (20 mM Tris-HCl (pH 7.6), 150 mM NaCl, 5 mM MgCl₂, 0.5% NP-40, 1 mM DTT) was performed 5 times, 40 µL of the luciferase substrate (Promega, E151A) diluted 2500-folds with the washing buffer was added to the wells and allowed to react for 5 minutes, and then the amount of luminescence was measured with a plate reader (PerkinElmer, Cat No. 5103-35).

### <Results>

The results are shown in Figure 1. All of Luc-CUG-PPR 1, Luc-CUG-PPR 2, Luc-CUG-PPR 3, and Luc-CUG-PPR 4 had binding ability against the CUG probe and no binding ability against the CAG probe. In other words, all of the PPR proteins had high binding specificity against the CUG repeat sequence.

### [Example 2: Effect of PPR on RNA aggregate formation in DM1 model cells]

PPR protein was allowed to act on DM1 model cells to measure the number of RNA aggregates with FISH (fluorescent in situ hybridization) method.

### <Experimental materials and methods>

### (1) DM1 model cells

A cell having the DMPK gene region comprising 800 CTG repeats introduced into a C2C12 cell (C2C12-DMPK800R, Nucleic Acids Research, 2014, 42(10), 6591-6602) was used as the DM1 cell model. C2C12-DMPK800 was produced with the following procedure. In other words, a plasmid (pLC16) comprising a sequence having 800 CTGs inserted into the 3' untranslated region of the DMPK gene and a plasmid that expresses PhiC31 integrase were both transfected to mouse myoblast strain C2C12 with Nucleofector (product name, from Lonza), and then stable expression strains were selected with a selection medium supplemented with puromycin. Subsequently, a plasmid that expresses Cre recombinase was transfected to the stable expression strain, and clones in which transcription of mRNA having 800 CUG repeats is induced were selected with a selection medium supplemented with hygromycin.

### (2) Production of PPR protein expression plasmid vector for cell transfection

A fusion gene in the order of green fluorescent protein mCLover3 gene, PPR protein gene, 3 x nucleus local signal, 3 x FLAG epitope tag was cloned into a mammal expression plasmid vector. The expression of the fusion gene is controlled by a CMV promoter and a SV40 poly-A signal. Cloning of the gene having the correct size was confirmed by PCR method, and the gene sequence was confirmed by sequencing. Moreover, as control, a fusion gene that does not comprise the PPR protein gene, i.e., a fusion gene of green fluorescent protein mCLover3 gene, 3 x nucleus local signal, and 3 x FLAG epitope tag, was prepared with the same method. These were respectively designated mCLo-CUG-PPR 1, mCLo-CUG-PPR 2, mCLo-CUG-PPR 3, mCLo-CUG-PPR 4, and mCLo-empty, and employed in subsequent tests. Note that the sequences of the PPR proteins (PPR 1 - PPR 4) that are encoded by each expression plasmid vector are identical to the sequences of each of the PPR proteins described in Table 1.

**[Table 6]**

| Table 2. Amino acid sequences encoded by the cloned genes | |
|---|---|
| Protein name | Seguence (PPR protein is underlined) |
| mCLo - CU G - PPR1 ( 17) (SEQ ID NO. 17) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTLID GLCKSGKIEEALKLFKEMEEKGITPSVVTYNTLIDGLCKAGRLDEAEELLEEME EKGIKPDVVTYTTLIDGLCKAGKVDEALELFDEMKERGIKPDVVTYNTLIDGL CKSGKIEEALKLFKEMEEKGITPSVVTYNTLIDGLCKAGRLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGRLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGRLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGRLDEAEELLEEMEEKGIKPDVVTY TTLIDGLCKAGKVDEALELFDEMKERGIKPDVVTYNTLIDGLCKSGKIEEALKL FKEMEEKGITPSVVTYNTLIDGLCKAGRLDEAEELLEEMEEKGIKPDVVTYTTL IDGLCKAGKVDEALELFDEMKERGIKPDELTYNTLISGLGKAGRARDPPVLSS PKKKRKVEDPKKKRKVEDPKKKRKVGSDYKDHDGDYKDHDIDYKDDDDK MAGR |
| mCLo - CU G - PPR2 (SQ ID ND. 18) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTNID QLCKSGKIEEALKLFKEMEEKGITPSVVTYNTLIDGLCKAGRLDEAEELLEEME EKGIKPDVVTYTTLIDGLCKAGKVDEALELFDEMKERGIKPDVVTYNTLIDGL CKSGKIEEALKLFKEMEEKGITPSVVTYNTLIDGLCKAGRLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGRLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGRLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGRLDEAEELLEEMEEKGIKPDVVTY |
| | TTLIDGLCKAGKVDEALELFDEMKERGIKPDVVTYNTLIDGLCKSGKIEEALKL FKEMEEKGITPSVVTYNTLIDGLCKAGRLDEAEELLEEMEEKGIKPDVVTYTTL IDGLCKAGKVDEALELFDEMKERGIKPDELTYNTLISGLGKAGRARDPPVLSS PKKKRKVEDPKKKRKVEDPKKKRKVGSDYKDHDGDYKDHDIDYKDDDDK MAGR |
| mCLo - CU G - PPR3 (SEQ ID NO. 19) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTNID QLCKSGKIEEALKLFKEMEEKGITPSVVTYNTLIDGLCKAGKLDEAEELLEEME EKGIKPDVVTYTTLIDGLCKAGKVDEALELFDEMKERGIKPDVVTYNTLIDGL CKSGKIEEALKLFKEMEEKGITPSVVTYNTLIDGLCKAGKLDEAEELLEEMEEK GIKPDVVTYTTLIDGLCKAGKVDEALELFDEMKERGIKPDVVTYNTLIDGLCK SGKIEEALKLFKEMEEKGITPSVVTYNTLIDGLCKAGKLDEAEELLEEMEEKGIK PDVVTYTTLIDGLCKAGKVDEALELFDEMKERGIKPDVVTYNTLIDGLCKSGK IEEALKLFKEMEEKGITPSVVTYNTLIDGLCKAGKLDEAEELLEEMEEKGIKPDV VTYTTLIDGLCKAGKVDEALELFDEMKERGIKPDVVTYNTLIDGLCKSGKIEEA LKLFKEMEEKGITPSVVTYNTLIDGLCKAGKLDEAEELLEEMEEKGIKPDVVTY TTLIDGLCKAGKVDEALELFDEMKERGIKPDVVTYNTLIDGLCKSGKIEEALKL FKEMEEKGITPSVVTYNTLIDGLCKAGKLDEAEELLEEMEEKGIKPDVVTYTTL IDGLCKAGKVDEALELFDEMKERGIKPDELTYNTLISGLGKAGRARDPPVLSS PKKKRKVEDPKKKRKVEDPKKKRKVGSDYKDHDGDYKDHDIDYKDDDDK MAGR |
| mCLo - CU G - PPR1 (SED ID NO. 20) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGNSVVTYNTNID QLCKSGRIEEALKLFKEMEEKGITPSVVTYNTLIDGLCKAGRLDEAEELLEEME EKGIKPDVVTYTTLIDGLCKAGRVDEALELFDEMKERGIKPDVVTYNTLIDGL CKSGRIEEALKLFKEMEEKGITPSVVTYNTLIDGLCKAGRLDEAEELLEEMEEK GIKPDVVTYTTLIDGLCKAGRVDEALELFDEMKERGIKPDVVTYNTLIDGLCKS GRIEEALKLFKEMEEKGITPSVVTYNTLIDGLCKAGRLDEAEELLEEMEEKGIKP DVVTYTTLIDGLCKAGRVDEALELFDEMKERGIKPDVVTYNTLIDGLCKSGRIE EALKLFKEMEEKGITPSVVTYNTLIDGLCKAGRLDEAEELLEEMEEKGIKPDVV |
| | TYTTLIDGLCKAGRVDEALELFDEMKERGIKPDVVTYNTLIDGLCKSGRIEEAL KLFKEMEEKGITPSVVTYNTLIDGLCKAGRLDEAEELLEEMEEKGIKPDVVTYT TLIDGLCKAGRVDEALELFDEMKERGIKPDVVTYNTLIDGLCKSGRIEEALKLF KEMEEKGITPSVVTYNTLIDGLCKAGRLDEAEELLEEMEEKGIKPDVVTYTTLI DGLCKAGRVDEALELFDEMKERGIKPDELTYNTLISGLGKAGRARDPPVLSSP KKKRKVEDPKKKRKVEDPKKKRKVGSDYKDHDGDYKDHDIDYKDDDDKM AGR |
| mCLo - em pty (SED ID NO. 21) | MAGVSKGEELFTGVVPILVELDGDVNGHKFSVRGEGEGDATNGKLTLKFICT TGKLPVPWPTLVTTFGYGVACFSRYPDHMKQHDFFKSAMPEGYVQERTISF KDDGTYKTRAEVKFEGDTLVNRIELKGIDFKEDGNILGHKLEYNFNSHYVYIT ADKQKNCIKANFKIRHNVEDGSVQLADHYQQNTPIGDGPVLLPDNHYLSHQ SKLSKDPNEKRDHMVLLEFVTAAGITHGMDELYKSRLHMGSGGLSSPKKKR KVEDPKKKRKVEDPKKKRKVGSDYKDHDGDYKDHDIDYKDDDDKMAGR |

### (3) Counting of RNA aggregate formation

DM1 model cells were cultured with DMEM medium containing 10% FBS and penicillin/streptomycin at 37°C under 5% CO₂ condition. Two hundred nanograms of the plasmid DNA constructed as above, 0.6 µL Fugene^{™}-HD (Promega, E2311), 200 µL Opti-MEM were mixed, the whole amount was added to the wells, cultured at 37°C under 5% CO₂ environment for 72 hours, and then fixed with 3% paraformaldehyde for 15 minutes at room temperature. After fixation, this was washed twice with PBS, and then permeabilized with PBS containing 0.5% TritonX-100 for 5 minutes. Subsequently, this was subjected to 10 minutes of prehybridization treatment with 2 x SSC buffer containing 30% formamide. Then, this was subjected to 1 hour of hybridization treatment at 37°C with 2 x SSC buffer containing 30% formamide, 2 µg/mL BSA, 66 µg/mL yeast tRNA, 2 mM vanadyl complex (vanadyl complex), and 1 ng/µL Texas Red CAG probe. After subjecting to 30 minutes of post hybridization treatment with 2 x SSC buffer containing 30% formamide at 42°C, this was washed once with 1 x SSC buffer, and then washed twice with PBS. After fixing with Vectashield with DAPI (product name, from Vector Laboratories), the number of RNA aggregates inside the nucleus was counted with a fluorescence microscope (Keyence PZ-9000).

### <Results>

The results are shown in Figure 2. As is clear from Figure 2, in DM1 model cells of the untreated group, the rate of RNA aggregate positive cells was greater than 40%, whereas in 4 experiment groups that were applied PPR protein, the rate of RNA aggregate positive cells was significantly reduced.

### [Example 3: Effect of PPR protein on splicing abnormality in DM1 model cells]

PPR protein was applied to DM1 model cells, and it was verified whether or not the splicing abnormality at exon 22 of the Atp2a1 gene improved.

### <Experimental materials and methods>

### (1) DM1 model cells

The same DM1 model cells as in Example 2 (C2C12-DMPK800R) was used.

### (2) PPR protein

The same expression plasmid vector as in Example 2 was used.

### (3) Quantification of splicing product

PPR protein expression plasmid vector was gene transferred into DM1 model cells via the same method as in Example 2, and after 72 hours, total RNA was extracted with RNeasy Mini Plus Kit (product name, from Qiagen). As control, DM1 model cells without gene transfer of PPR protein expression plasmid vector were employed, and total RNA was similarly extracted. Subsequently, cDNA was prepared with SuperScript III First Strand Symthesis System (from Invitrogen). After subjecting the cDNA to RNaseH treatment, RT-PCR was performed with the following Atp2a1 exon 22 RT primers. The RT-PCR product was subjected to electrophoresis with 2% agarose gel, stained with GelRed, and then normal type PCR products and abnormal type PCR products were each quantified with an image analyzer (ChemiDoc Touch Imaging System, from BioRad).
Atp2a1 exon 22 RT primers
Fw: GCTCATGGTCCTCAAGATCTCAC (SEQ ID NO. 22)
Rv: GGGTCAGTGCCTCAGCTTTG (SEQ ID NO. 23)

### <Results>

The results are shown in Figure 3. The lower portion is the electrophoresis result of the RT-PCR product, and the upper portion is a graph showing the proportion of the normal type in the RT-PCR product. In the PPR protein untreated group, the proportion of the abnormal type was larger than the normal type. By allowing PPR protein to act, the proportion of the normal type in DM1 model cells significantly increased, showing that the splicing abnormality improved.

### [Example 4: Effect of PPR protein on muscle differentiation abnormality in DM1 model cells]

PPR protein was allowed to act on DM1 model cells, and it was verified whether or not the muscle differentiation efficiency of DM1 model cells improved.

### <Experimental materials and methods>

### (1) DM1 model cells

The same DM1 model cells as in Example 2 (C2C12-DMPK800R) was used.

### (2) PPR protein

The same expression plasmid vector of PPR protein as in Example 2 was used.

### (3) Quantification of muscle differentiation efficiency

PPR protein expression plasmid vector was gene transferred into DM1 model cells via the same method as in Example 2. On the next day, the medium was exchanged to a differentiation medium, and 72 hours after transfection, nucleus staining by DAPI as well as antibody staining with anti-MyHC antibody were carried out. Antibody staining was first carried out, cells having 2 or more nuclei inside the cell were further set as myotube cells, and the total number of nuclei inside the myotube cells confirmed in the observation field was counted (hereinafter referred to as value A). Subsequently, the total number of nuclei in the whole field including not only the myotube cells in the above passage but also undifferentiated cells was counted (hereinafter referred to as value B). The Fusion index represents the percentage of value A divided by value B, and means the muscle differentiation efficiency.

### <Results>

The results are shown in Figure 4. The empty vector administration group (represented as "no" in the figure) showed a Fusion index of about 3. On the other hand, the PPR administration group all had a Fusion index of about 5 - 15, achieving recognition of significant improvement of muscle differentiation efficiency.

### [Example 5: Effect of PPR in DM1 model mice]

To DM1 model mice, AAV6 loaded with the CUG-PPR gene was administered in single dose into the anterior tibial muscle, and the effect on myotonia symptoms in the anterior tibial muscle, the effect on the splicing abnormality of the Atp2a1 gene and the skeletal muscle-type chloride channel (Clcn1) gene, as well as the number of RNA aggregate formation were verified.

### <Experimental materials and methods>

### (1) Animals used

As the DM1 model mice, HSA^{LR} mice (granted from Dr. Charles Thornton (University of Rochester) and bred in the laboratory animal facility at University of Osaka) were used. HSA^{LR} mouse is a transgenic animal having the CTG repeat sequence elongated 220 times at the 3' untranslated region of the hACTA gene (human gene constitutively expressed in myocytes) integrated into the genome, and the symptoms of DM1 appear when the mRNA having the CUG sequence elongated is expressed in myocytes (Science, 2000, 289 (5485), 1769-73). As control, wild-type mice (FVB/NJcl mice, purchased from CLEA Japan) were used. Fourteen weeks-old female and male mice were employed for the experiments.

### (2) PPR protein

### (Production of AAV vector)

To the multi-cloning site of an AAV vector (component of AAVpro^{™} Helper Free System (AAV6), Takara, 6651), a gene having green fluorescent protein mCLover3, PPR protein, 3 x nuclear localization signal, and 3 x FLAG epitope tag fused in order was inserted. Verification of the expression size of the constructed gene by PCR, and verification of the inserted sequence by sequencing was performed. The expression of the fusion gene is controlled by the CMV promoter and the Human growth hormone polyA signal.

### (Preparation of AAV vector)

Twenty micrograms each of the AAV vector constructed in the preceding section, the pRC6 vector and the phelper vector (both components of AAVpro^{™} Helper Free System (AAV6), Takara, 6651) were gene transferred into 2.5 x 10⁸ cells of the AAVpro^{™} HEK293T cell line (Takara, 632273) with Polyethylenimine, Linear, MW 25000 (Polysciences, Inc., 23966-1). Three days after transfection, AAV producer cells were obtained. AAVpro^{™} Purification Kit Maxi (all serotypes) (Takara, 6666) was employed for AAV purification. Titration was carried out with AAVpro titration kit for real time PCR ver. 2 (Takara, 6233), and this was diluted to 5 x 10¹¹ vg/mL with D-PBS and set as the administration solution. These were respectively designated AAV6-mCLo-CUG-PPR 1, AAV6-mCLo-CUG-PPR 2, and AAV6-mCLo-empty, and employed in subsequent tests. Note that the sequences of the PPR proteins (PPR 1, PPR 2) that are encoded by each AAV vector are identical to the sequences of each of the PPR proteins described in Table 1.

### (3) Grouping and administration schedule

The four groups of the AAV6-mCLo-CUG-PPR 1 administration DM1 group, the AAV6-mCLo-CUG-PPR 2 administration DM1 group, the AAV6-mCLo-empty administration DM1 group, and the PBS administration DM1 group were set (n = 5 for each group (left and right limbs of 3 males and 2 females were used). The dosage of AAV was 1 x 10¹⁰ vg/leg and administered in single dose. To the PBS administration DM1 group, the same volume of PBS was administered in single dose.

### (4) Counting of RNA aggregate formation

Fifty-six days after the final administration, the anterior tibial muscle was collected from the mice and sections were produced. These were washed twice with PBS, and then permeabilized with PBS containing 0.5% TritonX-100 for 5 minutes. Subsequently, these were subjected to 10 minutes of prehybridization treatment with 2 x SSC buffer containing 30% formamide. Then, these were subjected to 1 hour of hybridization treatment at 37°C with 2 x SSC buffer containing 30% formamide, 2 µg/mL BSA, 66 µg/mL yeast tRNA, 2 mM vanadyl complex (vanadyl complex), and 1 ng/µL Texas Red CAG probe. After subjecting to 30 minutes of post hybridization treatment with 2 x SSC buffer containing 30% formamide at 42°C, these were washed once with 1 x SSC buffer, and then washed twice with PBS. After fixing with Vectashield with DAPI (product name, from Vector Laboratories), the number of RNA aggregates inside the nucleus was counted with a fluorescence microscope (Keyence PZ-9000).

### (5) Quantification of splicing product

Fifty-six days after the final administration, the anterior tibial muscle was collected from the mice. After extracting total RNA with TRI Reagent (product name, MRC), cDNA was prepared with SuperScript III First Strand Symthesis System (from Invitrogen). After subjecting the cDNA to RNaseH treatment, RT-PCR was performed with Atp2a1 exon 22 RT primers similar to Example 2 and the following Clcn1 exon 7a RT primers. The RT-PCR product was subjected to electrophoresis with 2% agarose gel, stained with GelRed, and then normal type PCR products and abnormal type PCR products were each quantified with an image analyzer (ChemiDoc Touch Imaging System, from BioRad), and the proportion of the normal type in the RT-PCR product was calculated. T-test was employed for statistical analysis.
Clcn1 exon 7a RT primers
Fw: TGAAGGAATACCTCACACTCAAGG (SEQ ID NO. 24)
Rv: CACGGAACACAAAGGCACTG (SEQ ID NO. 25)

### (6) Measurement of myotonia

Fifty-six days after the final administration, the electrical myotonia phenomenon of the mouse anterior tibial muscle was analyzed with needle electromyography under anesthesia. Specifically, the occurrence frequency of electrical myotonia phenomenon when a needle electrode was inserted 20 times into the mouse anterior tibial muscle was evaluated in the following 4 stages.
Severity 0: No electrical myotonia phenomenon is seen at all.
Severity 1: Electrical myotonia phenomenon is seen in less than 50% of total.
Severity 2: Electrical myotonia phenomenon is seen in 50% or more and less than 90% of total.
Severity 3: Electrical myotonia phenomenon is seen in 90% or more.

### <Results>

The results of RNA-foci analysis are shown in Figure 5. In the PBS administration group and the AAV6-mCLo-empty administration group, the positive cell rate of the RNA-foci was shown to be about 30%. On the other hand, for the AAV6-mCLo-CUG-PPR 1 and the AAV6-mCLo-CUG-PPR 2 administration groups, significant decrease in the RNA-foci positive rate was recognized.

The evaluation results of splicing abnormality are shown in Figure 6. In regard to Atp2a1, in the PBS administration DM1 group and the AAV6-mCLo-Empty administration MyoD group, the proportion of the normal type was shown to be about 60%. In the AAV6-mCLo-CUG-PPR 1 administration DM1 group and the AAV6-mCLo-CUG-PPR 2 administration DM1 group, it became clear from the significant increase in the proportion of the normal type RT-PCR product that the splicing abnormality of the Atp2a1 gene is improved by PPR protein administration. Similarly for Clcn1, it was recognized that the splicing abnormality of the Clcn1 gene was improved by PPR administration.

The analytical results of myotonia are shown in Figure 7. For the PBS administration group and the AAV6-mCLo-empty administration group, myotonia of Severity 2 was detected in all five out of five mice. For both the AAV6-mCLo-CUG-PPR 1 administration DM1 group and the AAV6-mCLo-CUG-PPR 2 administration DM1 group, two out of five mice showed Severity 1 and the remaining three mice showed Severity 2. Moreover, the improved two cases were from the experiment group with high improvement effect for Clcn1 splicing abnormality (Figure 8).

From the above results, it was shown that splicing abnormality based on abnormal elongation of the CTG repeat sequence is improved by the present invention, and as a result, myotonia symptoms in DM1 is improved.

### [Example 6: Dose-dependency test of drug effect with M1 model mice]

In order to evaluate the dose-dependency of the drug effect employing DM1 model mice, AAV9 loaded with the CUG-PPR gene was administered in single dose to DM1 model mice in the tail vein at each amounts of 3 x 10¹³, 1 x 10¹⁴, and 3 x 10¹⁴ vg/kg, and the effect on myotonia symptoms in the femoral muscle, the effect on the splicing abnormality of the Atp2a1 gene and the skeletal muscle-type chloride channel (Clcn1) gene, as well as the number of RNA aggregate formation were verified.

### <Experimental materials and methods>

### (1) Animals used

As the DM1 model mice, HSA^{LR} mice (granted from Dr. Charles Thornton (University of Rochester) and bred in the laboratory animal facility at University of Osaka) were used. HSA^{LR} mouse is a transgenic animal having the CTG repeat sequence elongated 220 times at the 3' untranslated region of the hACTA gene (human gene constitutively expressed in myocytes) integrated into the genome, and the symptoms of DM1 appear when the mRNA having the CUG sequence elongated is expressed in myocytes (Science, 2000, 289 (5485), 1769-73). As control, wild-type mice (FVB/NJcl mice, purchased from CLEA Japan) were used. Fourteen weeks-old female and male mice were employed for the experiments.

### (2) PPR protein

### (Production of AAV vector)

To the multi-cloning site of a pAAV-CMV vector (component of AAVpro^{™} Helper Free System, Takara, 6651), a gene having PPR protein and 3 x nuclear localization signal fused in order was inserted. Verification of the expression size of the constructed gene by PCR, and verification of the inserted sequence by sequencing was performed. The expression of the fusion gene is controlled by the CMV promoter and the Human growth hormone polyA signal.

### (Preparation of AAV vector)

The production of the AAV vector constructed in the preceding section was outsourced to SignaGen Laboratories (hereinafter abbreviated as SG Company). Packaging was carried out by co-transfecting the packaging cell strain with the Rep/Cap plasmid and the Helper plasmid from SG Company and the AAV vector sent from the inventors' company. The cell homogenate comprising the packaged AAV was purified by density gradient centrifugation and titrated. The AAV obtained was 1.16 x 10¹⁴ vg/ml and the volume was 5 ml. This was employed in the subsequent experiment as AAV9-CUG-PPR 1. Note that the sequences of the PPR proteins (PPR 1) encoded in each AAV vector are identical to the sequences of the PPR proteins described in Table 1.

### (3) Grouping and administration schedule

The four groups of the AAV9-CUG-PPR 1 low dose administration group (hereinafter abbreviated as Low dose: LD), the AAV9-CUG-PPR 1 medium dose administration group (hereinafter abbreviated as Middle dose: MD), the AAV9-CUG-PPR 1 high dose administration group (hereinafter abbreviated as High dose: HD), and the PBS administration group (hereinafter abbreviated as PBS) were set. n = 5 for each group (3 male and 2 female mice). The dosage of AAV was 3 x 10¹³ vg/kg for LD, 1 x 10¹⁴ vg/kg for MD, and 3 x 10¹⁴ vg/kg for HD, and administered in single dose in the tail vein. To the PBS administration group, the same volume of PBS was administered in single dose.

### (4) Counting of RNA aggregate formation

Twenty-eight days after the final administration, the femoral muscle was collected from the mice and sections were produced. These were washed twice with PBS, and then permeabilized with PBS containing 0.5% TritonX-100 for 5 minutes. Subsequently, these were subjected to 10 minutes of prehybridization treatment with 2 x SSC buffer containing 30% formamide. Then, these were subjected to 1 hour of hybridization treatment at 37°C with 2 x SSC buffer containing 30% formamide, 2 µg/mL BSA, 66 µg/mL yeast tRNA, 2 mM vanadyl complex (vanadyl complex), and 1 ng/µL Texas Red CAG probe. After subjecting to 30 minutes of post hybridization treatment with 2 x SSC buffer containing 30% formamide at 42°C, these were washed once with 1 x SSC buffer, and then washed twice with PBS. After fixing with Vectashield with DAPI (product name, from Vector Laboratories), the number of RNA aggregates inside the nucleus was counted with a fluorescence microscope (Keyence PZ-9000) .

### (5) Quantification of splicing product

Twenty-eight days after the final administration, the femoral muscle was collected from the mice. After extracting total RNA with TRI Reagent^{™} (MRC), cDNA was prepared with SuperScript III First Strand Symthesis System (from Invitrogen). After subjecting the cDNA to RNaseH treatment, RT-PCR was performed with Atp2a1 exon 22 RT primers similar to Example 2 and the following Clcn1 exon 7a RT primers. The RT-PCR product was subjected to electrophoresis with 2% agarose gel, stained with GelRed, and then normal type PCR products and abnormal type PCR products were each quantified with an image analyzer (ChemiDoc Touch Imaging System, from BioRad), and the proportion of the normal type in the RT-PCR product was calculated. T-test was employed for statistical analysis.

### Clcn1 exon 7a RT primers

Fw: TGAAGGAATACCTCACACTCAAGG (SEQ ID NO. 24)
Rv: CACGGAACACAAAGGCACTG (SEQ ID NO. 25)

### (6) Measurement of myotonia

Twenty-eight days after the final administration, the electrical myotonia phenomenon of the mouse femoral muscle was analyzed with needle electromyography under anesthesia. Specifically, the occurrence frequency of electrical myotonia phenomenon when a needle electrode was inserted 20 times into the mouse femoral muscle was evaluated in the following 4 stages. Severity 0: No electrical myotonia phenomenon is seen at all. Severity 1: Electrical myotonia phenomenon is seen in less than 50% of total.
Severity 2: Electrical myotonia phenomenon is seen in 50% or more and less than 90% of total.
Severity 3: Electrical myotonia phenomenon is seen in 90% or more.

### (7) Measurement of amount of PPR mRNA expression in AAV administration mouse tissue

The resected femoral muscle organ fragment was wet with 800 µL of the TRIzol Reagent. Two homogenization beads (TOMY, SUB-30) were added, and the organ was homogenized with a beads cell homogenizing device Micro Smash (TOMY SEIKO, MS-100R). Six hundred microliters of the supernatant obtained after centrifugation at 4°C, 15,000 g for 5 minutes were collected, 160 µL of chloroform solution was added, and this was mixed by inversion at room temperature for 5 minutes. Then, this was centrifugated at 4°C, 15,000 g for 5 minutes, and separated into the aqueous phase and the organic phase. Three hundred microliters of the aqueous phase were collected, and this was subjected to nucleic acid purification device Maxwell RSC Instrument to obtain a purified total-RNA product. The concentration and the purity of the RNA was confirmed by carrying out absorbance measurement with NanoDrop 8000. In order to verify the degree of degradation of the RNA obtained, 20 - 250 ng/µL of the total-RNA was used to perform electrophoresis by LabChip GX Touch HT (PerkinElmer, CLS137031J), and it was confirmed from the band strength ratio of 28S and 18S rRNA that degradation had not proceeded. For cDNA synthesis, 20 - 100 ng/µL of the RNA obtained was subjected to reverse transcription reaction according to the product protocol of SuperScript III Reverse Transcriptase (Thermo Fisher Scientific (Life Technologies), 18080085). For the amounts of PPR mRNA and GAPDH mRNA, the cDNA produced, Brilliant III Ultra-Fast SYBR Green QPCR Master Mix (Agilent, 600882), and the primers shown below were mixed according to manual, and then amplified with real-time PCR system Aria MX (Agilent) to perform quantitative analysis.

### Primers for PPR quantitative PCR

Fw: GATGAGGCTTTGGAACTGTTTG (SEQ ID NO. 26)
Rv: TCTCTGGCTCTGCCGGCCTTGC (SEQ ID NO. 27)

### Primers for GAPDH quantitative PCR

Fw: ATCATCCCTGCCTCTACTGG (SEQ ID NO. 28)
Rv: CTGCTTCACCACCTTCTTGA (SEQ ID NO. 29)

### <Results>

The results of RNA-foci analysis are shown in Figure 9. The femoral muscle organ sections obtained were employed to carry out RNA-FISH, and the RNA-foci formation suppression effect by AAV9-CUG-PPR 1 was verified. As a result, dose-dependent suppression effect on the formation of RNA-foci was confirmed in the AAV9-CUG-PPR 1 administration group. In regard to the RNA-foci positive cell rate, formation suppression effect observed were 39% for the PBS administration group, 18% for the AAV9-CUG-PPR 1 low dose group, 23% for the AAV9-CUG-PPR 1 medium dose group, and 17% for the AAV9-CUG-PPR 1 high dose administration group.

The evaluation results of splicing abnormality are shown in Figure 10. Total RNA was collected from the organ obtained, reverse transcription PCR was performed, and abnormal splicing of the Clcn1 gene and the Atp2a1 gene were evaluated. The abnormal splicing phenomenon of Clcn1 is the Cl channel which is known to be the direct cause of myotonic discharge phenomenon. Atp2a1 is a Ca transporter likewise related to myotonic discharge, and is known to be a sensitive marker gene. As a result of splicing analysis, in the detection system of Clcn1, the PBS administration group showed 58% normal splicing, whereas the AAV9-CUG-PPR 1 low dose group showed 72%, the AAV9-CUG-PPR 1 medium dose group showed 78%, and the AAV9-CUG-PPR 1 high dose group showed 82%, confirming a dose-dependent splicing improvement. In the detection system of Atp2a1, the PBS administration group showed 25% normal splicing, whereas the AAV9-CUG-PPR 1 low dose group showed 56%, the AAV9-CUG-PPR 1 medium dose group showed 60%, and the AAV9-CUG-PPR 1 high dose group showed 64%, showing a dose-dependent splicing improvement.

The decrease improvement effect of myotonic discharge by PPR was represented by a score in 4 stages based on the number of myotonic discharge. In other words, Score 0 means detection of myotonic discharge in 0 out of 20 times, Score 1 means 1-9 times out of 20, Score 2 means 10 - 19 times out of 20, and Score 3 means 20 out of 20 times. As a result of myotonic discharge analysis employing the femoral muscle, the PBS administration group showed Score 3, the AAV9-CUG-PPR 1 low dose group showed Score 3 in 1/5 case and Score 2 in 4/5 cases, the medium dose group showed Score 2 in 4/5 cases and Score 1 in 1/5 case, and the low dose group showed improvement to Score 2 in 3/5 cases and Score 1 in 2/5 cases (Figure 11).

In order to investigate the pharmacokinetics, total RNA was extracted from the analytical organ femoral muscle and quantitative PCR of PPR mRNA was carried out. As a result of RT-qPCR, dose-dependent expression of PPR mRNA was confirmed (Figure 12). For the expression amount of PPR mRNA for each group, the relative value when the value of the individual with the lowest signal in the AAV9-CUG-PPR 1 low dose administration group was set as 1 using GAPDH as the internal standard was used. Taking the median of each group, the PBS administration group was 0.2, the low dose administration group was 0.7, the medium dose administration group was 1.8, and the high dose administration group was 2.6, confirming a dose-dependent PPR expression (Figure 12) .

From the above results, dose-dependent suppression of myotonic discharge of AAV9-CUG-PPR 1, RNA-foci formation suppression effect, and abnormal splicing improvement effect were confirmed.

### [Example 7: Time base change in drug effect in DM1 model mice]

In order to evaluate the time base change in the drug effect employing DM1 model mice, AAV9 loaded with the CUG-PPR gene was administered in single dose to DM1 model mice in the tail vein, and the effect on myotonia symptoms in the femoral muscle at each time point, the effect on the splicing abnormality of the Atp2a1 gene and the skeletal muscle-type chloride channel (Clcn1) gene, as well as the number of RNA aggregate formation were verified.

### <Experimental materials and methods>

### (1) Animals used

As the DM1 model mice, HSA^{LR} mice (granted from Dr. Charles Thornton (University of Rochester) and bred in the laboratory animal facility at University of Osaka) were used. HSA^{LR} mouse is a transgenic animal having the CTG repeat sequence elongated 220 times at the 3' untranslated region of the hACTA gene (human gene constitutively expressed in myocytes) integrated into the genome, and the symptoms of DM1 appear when the mRNA having the CUG sequence elongated is expressed in myocytes (Science, 2000, 289 (5485), 1769-73). As control, wild-type mice (FVB/NJcl mice, purchased from CLEA Japan) were used. Twelve weeks-old female and male mice were employed for the experiments.

### (2) PPR protein

### (Production of AAV vector)

To the multi-cloning site of a pAAV-CMV vector (component of AAVpro^{™} Helper Free System, Takara, 6651), a gene having PPR protein and 3 x nuclear localization signal fused in order was inserted. Verification of the expression size of the constructed gene by PCR, and verification of the inserted sequence by sequencing was performed. The expression of the fusion gene is controlled by the CMV promoter and the Human growth hormone polyA signal.

### (Preparation of AAV vector)

The production of the AAV vector constructed in the preceding section was consigned to SignaGen Laboratories (hereinafter abbreviated as SG Company). Packaging was carried out by co-transfecting the packaging cell strain with the Rep/Cap plasmid and the Helper plasmid from SG Company and the AAV vector sent from the inventors' company. The cell homogenate comprising the packaged AAV was purified by density gradient centrifugation and titrated. The AAV obtained was 1.16 x 10¹⁴ vg/ml and the volume was 5 ml. This was employed in the subsequent experiment as AAV9-CUG-PPR 1. Note that the sequences of the PPR proteins (PPR 1) encoded in each AAV vector are identical to the sequences of the PPR proteins described in Table 1.

### (3) Grouping and administration schedule

The four groups of 14 days after administration (2 w), 28 days after administration (4 w), 56 days after administration (8 w), and 112 days after administration (16 w) of AAV9-CUG-PPR 1 were set. n = 5 for each group (2 males and 3 females) of mice was used. The dosage of AAV was 3 x 10¹⁴ vg/kg, and administered in single dose in the tail vein.

### (4) Counting of RNA aggregate formation

14, 28, 56, or 112 days after the final administration, the femoral muscle was collected from the mice and sections were produced. These were washed twice with PBS, and then permeabilized with PBS containing 0.5% TritonX-100 for 5 minutes. Subsequently, these were subjected to 10 minutes of prehybridization treatment with 2 x SSC buffer containing 30% formamide. Then, these were subjected to 1 hour of hybridization treatment at 37°C with 2 x SSC buffer containing 30% formamide, 2 µg/mL BSA, 66 µg/mL yeast tRNA, 2 mM vanadyl complex (vanadyl complex), and 1 ng/µL Texas Red CAG probe. After subjecting to 30 minutes of post hybridization treatment with 2 x SSC buffer containing 30% formamide at 42°C, these were washed once with 1 x SSC buffer, and then washed twice with PBS. After fixing with Vectashield with DAPI (product name, from Vector Laboratories), the number of RNA aggregates inside the nucleus was counted with a fluorescence microscope (Keyence PZ-9000) .

### (5) Quantification of splicing product

14, 28, 56, or 112 days after the final administration, the femoral muscle was collected from the mice. After extracting total RNA with TRI Reagent^{™} (MRC), cDNA was prepared with SuperScript III First Strand Symthesis System (from Invitrogen). After subjecting the cDNA to RNaseH treatment, RT-PCR was performed with Atp2a1 exon 22 RT primers similar to Example 2 and the following Clcn1 exon 7a RT primers. The RT-PCR product was subjected to electrophoresis with 2% agarose gel, stained with GelRed, and then normal type PCR products and abnormal type PCR products were each quantified with an image analyzer (ChemiDoc Touch Imaging System, from BioRad), and the proportion of the normal type in the RT-PCR product was calculated. T-test was employed for statistical analysis.

### Clcn1 exon 7a RT primers

Fw: TGAAGGAATACCTCACACTCAAGG (SEQ ID NO. 24)
Rv: CACGGAACACAAAGGCACTG (SEQ ID NO. 25)

### (6) Measurement of myotonia

Immediately before autopsy, the electrical myotonia phenomenon of the mouse femoral muscle was analyzed with needle electromyography under anesthesia. Specifically, the occurrence frequency of electrical myotonia phenomenon when a needle electrode was inserted 20 times into the mouse femoral muscle was evaluated in the following 4 stages.
Severity 0: No electrical myotonia phenomenon is seen at all.
Severity 1: Electrical myotonia phenomenon is seen in less than 50% of total.
Severity 2: Electrical myotonia phenomenon is seen in 50% or more and less than 90% of total.
Severity 3: Electrical myotonia phenomenon is seen in 90% or more.

### <Results>

The results of RNA-foci analysis are shown in Figure 13. The femoral muscle organ sections obtained were employed to carry out RNA-FISH, and the RNA-foci formation suppression effect by AAV9-CUG-PPR 1 was verified. As a result, dose-dependent suppression effect on the formation of RNA-foci was confirmed in the AAV9-CUG-PPR 1 administration group. The value of the PBS administration group employed in the dose-dependency test of Example 6 was used as control. In regard to the RNA-foci positive cell rate, formation suppression effect confirmed were 39% for the control group, 36% for the Day 14 after administration of AAV9-CUG-PPR 1 group, 23% for the Day 28 after administration group, 19% for the Day 56 after administration group, and 18% for the Day 112 after administration group (Figure 13).

The evaluation results of splicing abnormality are shown in Figure 14. Total RNA was collected from the organ obtained, reverse transcription PCR was performed, and abnormal splicing of the Clcn1 gene and the Atp2a1 gene were evaluated. The abnormal splicing phenomenon of Clcn1 is the Cl channel which is known to be the direct cause of myotonic discharge phenomenon. Atp2a1 is a Ca transporter likewise related to myotonic discharge, and is known to be a sensitive marker gene. As a result of splicing analysis, in the detection system of Clcn1, the PBS administration group shown in Example 6 showed 58% normal splicing, whereas the experiment group of Day 14 after administration of AAV9-CUG-PPR 1 showed 62%, Day 28 after administration of AAV9-CUG-PPR 1 showed 76%, and Day 56 after administration of AAV9-CUG-PPR 1 showed 79%, and 80% splicing improvement was confirmed for Day 112 after administration of AAV9-CUG-PPR 1. In the detection system of Atp2a1, the PBS administration group shown in Example 6 showed 25% normal splicing, whereas the experiment group of Day 14 after administration of AAV9-CUG-PPR 1 showed 30%, Day 28 after administration of AAV9-CUG-PPR 1 showed 63%, and Day 56 after administration of AAV9-CUG-PPR 1 showed 76%, and 75% splicing improvement was confirmed for Day 112 after administration of AAV9-CUG-PPR 1 (Figure 14).

The decrease improvement effect of myotonic discharge by PPR was represented by a score in 4 stages based on the number of myotonic discharge. In other words, Score 0 means detection of myotonic discharge in 0 out of 20 times, Score 1 means 1-9 times out of 20, Score 2 means 10 - 19 times out of 20, and Score 3 means 20 out of 20 times. As a result of myotonic discharge analysis employing the femoral muscle, the PBS administration group shown in Example 6 showed Score 3, the experiment group of Day 14 after administration of AAV9-CUG-PPR 1 showed Score 3 in 4/5 cases and Score 2 in 1/5 case, the experiment group of Day 28 after administration of AAV9-CUG-PPR 1 showed Score 2 in 5/5 cases, the experiment group of Day 56 after administration of AAV9-CUG-PPR 1 showed Score 2 in 3/5 cases and Score 1 in 2/5 cases, and the experiment group of Day 112 after administration showed Score 2 in 3/5 cases and Score 1 in 2/5 cases (Figure 15).

In order to investigate the pharmacokinetics, total RNA was extracted from the analytical organ femoral muscle and quantitative PCR of PPR mRNA was carried out. As a result of RT-qPCR, continuous expression of PPR was confirmed even at Day 112 after administration (data not shown).

From the above results, it was confirmed that AAV9-CUG-PPR 1-administered mice showed improvement of drug effect until 14 days, 28 days, and 56 days after administration, and the drug effect still continued at Day 112 after administration.

### [Example 8: Comprehensive improvement of DM1-related splicing abnormality by AAV9-CUG-PPR 1 administration]

### <Experimental materials and methods>

### (1) Analytical organ

To HSALR mice (14 weeks-old), AAV9-CUG-PPR 1 was administered in single dose at 3 x 10¹⁴ vg/kg in the tail vein, and the femoral muscle tissue fragment resected 8 weeks after administration (Day 56) was set as the PPR therapy group. The same volume of PBS was administered in single dose, and the resected femoral muscle was set as the PPR untreated group. The femoral muscle tissue fragment of a wild-type FVB/NJcl mouse having similar genetic background was employed as the wild-type group.

### (2) Analysis of abnormal splicing events

Following the total RNA extraction method described in Example 6, total-RNA was extracted from the femoral muscle of the PPR therapy group and the PPR untreated group and the wild-type group, and necessary amounts were sent to GENEZWIZ to carry out RNA Sequence (Illumina Hiseq 2 x 150 bp sequence). In the RNA sent, ribosomal RNA in the total RNA was removed by Poly A selection method. RNA was reverse transcripted, cDNA was synthesized, and a library with adaptor was prepared. Sequencing was performed, and adaptor trimming was carried out on the lead information obtained with Trimmomatic (v0.33) 59. Employing GRCm39 (obtained from Ensemble) as the reference genome, alignment was performed on the lead sequence after trimming with STAR (v2.7.9a). Specification of splicing events was detected with rMATS (v3.2.5) based on the STAR-aligned file (.bam). For specification of the varied splicing events, comparison of the wild-type group and the PPR untreated group (HSALR mice administered with PBS) was performed, the presence or absence of variation was judged with "p-adjusted-value" and "InclLevelDifference" as indicators, and only the candidates that were defined to be varied were extracted. To the varied splicing events, cutoff with a threshold of FDR (False Discovery Rate)-adjusted P value < 0.05 was performed and employed for a comparison test between groups. The Percent spliced-in index (PSI) was defined as the proportion of the amount of spliced-in transcript against the total lead number of the target gene. Metascape (https://metascape.org/gp/index.html#/main/step1) was used for gene ontology analysis.

### <Results>

Total RNA was extracted from the femoral muscle resected from wild-type mice (14 weeks-old) described in the preceding section (hereinafter referred to as the wild-type group), the femoral muscle resected 8 weeks after PBS administration to HSALR mice (hereinafter referred to as the PPR untreated group), and further the femoral muscle resected 8 weeks (Day 56) after AAV9-CUG-PPR 1 administration (hereinafter the PPR treatment group), and exhaustive analysis of expressed RNA was carried out by next-generation sequencing technology.

After mapping of the lead sequence obtained, the number of leads of adjacent exon sequences were compared, PSI (percent spliced-in index) was calculated, and the inclusion levels of each exon of each gene were calculated. Among the PSI of the wild-type and the PBS administration groups, those having the difference of 0.2 or more were set to be abnormal splicing events in HSALR mice (n = 455, Figure 16a left). As a result of comparing the PSI of the same splicing events at Week 8 after administration of AAV9-CUG-PPR 1 and the PSI in WT (Figure 16a right), it was found that PSI had generally recovered.

In the following literature, improvement was also seen in splicing events reported to have splicing abnormality in HSA-LR other than Atp2a1 or Clcn1 (Figure 16b).

### Reference literature: Matthew K Tanner et al. Nucleic Acids Research, Volume 49, Issue 4(2021)

Gene ontology analysis was performed on the gene group with abnormal splicing, and they were the terms related to muscle function or muscle differentiation. As a result of analyzing the number of genes in each gene ontology that achieved recognition of improvement, it was found that genes achieving recognition of strong improvement (greater than 50%) was about 40%, coming to a total of 90% including those achieving recognition of improvement (greater than 20%) (Figure 16c) .

From these, it was found that various splicing abnormalities are improved by administration of AAV9-CUG-PPR 1.

### [Example 9: Analysis of PPR mRNA expression amount in each organ of normal mice that were applied AAV9-CUG-PPR 1]

### <Experimental materials and methods>

### (1) Analytical organ

Four organ fragments (heart, femoral muscle, gastrocnemius muscle, and anterior tibial muscle) resected from normal mice iv-administered with AAV9-CUG-PPR 1 (administration dosage: 1 × 10¹³ vg/kg, 3 × 10¹³ vg/kg, or 3 × 10¹⁴ vg/kg) were used.

### (2) Administration schedule and autopsy timing

Eight weeks-old normal female mice were employed. Administration dosages were 1 × 10¹³ vg/kg, 3 × 10¹³ vg/kg, or 3 × 10¹⁴ vg/kg and administration was performed in the tail vein, and the volume was set to be 10 µL/g. Observation duration was set to be 28 days after administration. The heart, the femoral muscle, the gastrocnemius muscle, and the anterior tibial muscle was resected from each mouse individual to perform analysis of PPR mRNA.

### (3) Measurement of amount of PPR mRNA expression in AAV administration mouse tissue

Total-RNA was extracted from the resected organ fragments, cDNA was synthesized, and quantitative PCR was carried out. First, 50 mg (± 5 mg) fragments of the organ fragments obtained in the preceding section were wet with 800 µL of the TRIzol Reagent. Two homogenization beads (TOMY, SUB-30) were added, and the organs were homogenized with a beads cell homogenizing device Micro Smash (TOMY SEIKO, MS-100R), and this was centrifugated at 4°C, 15,000 g for 5 minutes. Six hundred microliters of the supernatant obtained was collected, 160 µL of chloroform solution was added, and this was mixed by inversion at room temperature for 5 minutes. Then, this was centrifugated at 4°C, 15,000 g for 5 minutes, and separated into the aqueous phase and the organic phase. Three hundred microliters of the aqueous phase were collected, and this was subjected to nucleic acid purification device Maxwell RSC Instrument to obtain a purified total-RNA product. The concentration and the purity of the RNA was confirmed by carrying out absorbance measurement with NanoDrop 8000. In order to verify the degree of degradation of the RNA obtained, 20 - 250 ng/µL of the total-RNA was used to perform electrophoresis by LabChip GX Touch HT (PerkinElmer, CLS137031J), and it was confirmed from the band strength ratio of 28S and 18S rRNA that degradation had not proceeded.

For cDNA synthesis, 20 - 100 ng/µL of the RNA obtained was subjected to reverse transcription reaction according to the product protocol of SuperScript III Reverse Transcriptase (Thermo Fisher Scientific (Life Technologies), 18080085).

Quantitative PCR was carried out with real-time PCR system Aria MX (Agilent) according to the general manual of Brilliant III Ultra-Fast SYBR Green QPCR Master Mix (Agilent, 600882).

### Primers for PPR quantitative PCR

Fw: GATGAGGCTTTGGAACTGTTTG (SEQ ID NO. 26)
Rv: TCTCTGGCTCTGCCGGCCTTGC (SEQ ID NO. 27)

### Primers for GAPDH quantitative PCR

Fw: ATCATCCCTGCCTCTACTGG (SEQ ID NO. 28)
Rv: CTGCTTCACCACCTTCTTGA (SEQ ID NO. 29)

### <Results>

The results of quantitative PCR are shown in Figure 17. As a result of quantitative PCR, a rise in dose-dependent PPR expression amount in each tissue was confirmed, and a maximum expression amount was shown in the maximally administered 3 x 10¹⁴ vg/kg administration group.

From the above, it was shown that AAV9-CUG-PPR 1 can be systematically delivered and expressed, and suggests that it is effective for DM1 which is a multi-organ disease that affects the heart in addition to skeletal muscles and smooth muscles.

### [Example 10: Analysis of PPR mRNA expression persistency in each organ of normal mice that were applied AAV9-CUG-PPR 1]

### <Experimental materials and methods>

### (1) Analytical organ

Four organ fragments (heart, femoral muscle, gastrocnemius muscle, and anterior tibial muscle) resected from normal mice iv-administered with AAV9-CUG-PPR 1 (administration dosage: 1 × 10¹⁴ vg/kg) were used.

### (2) Administration schedule and autopsy timing

Eight weeks-old normal male mice were employed. Administration dosage was 1 × 10¹⁴ vg/kg and administration was performed in the tail vein, and the volume was set to be 10 µL/g. Observation duration was set to be Day 14 after administration, Day 28 after administration, Day 56 after administration, Day 112 after administration, and Day 182 after administration. The heart, the femoral muscle, the gastrocnemius muscle, and the anterior tibial muscle was resected from each mouse individual to perform analysis of PPR mRNA.

### (3) Measurement of amount of PPR mRNA expression in AAV administration mouse tissue

Total-RNA was extracted from the resected organ fragments, cDNA was synthesized, and quantitative PCR was carried out. First, 50 mg (± 5 mg) fragments of the organ fragments obtained in the preceding section were wet with 800 µL of the TRIzol Reagent. Two homogenization beads (TOMY, SUB-30) were added, and the organs were homogenized with a beads cell homogenizing device Micro Smash (TOMY SEIKO, MS-100R). Six hundred microliters of the supernatant obtained after centrifugation at 4°C, 15,000 g for 5 minutes was collected, 160 µL of chloroform solution was added, and this was mixed by inversion at room temperature for 5 minutes. Then, this was centrifugated at 4°C, 15,000 g for 5 minutes, and separated into the aqueous phase and the organic phase. Three hundred microliters of the aqueous phase were collected, and this was subjected to nucleic acid purification device Maxwell RSC Instrument to obtain a purified total-RNA product. The concentration and the purity of the RNA was confirmed by carrying out absorbance measurement with NanoDrop 8000. In order to verify the degree of degradation of the RNA obtained, 20 - 250 ng/µL of the total-RNA was used to perform electrophoresis by LabChip GX Touch HT (PerkinElmer, CLS137031J), and it was confirmed from the band strength ratio of 28S and 18S rRNA that degradation had not proceeded. For cDNA synthesis, 20 - 100 ng/µL of the RNA obtained was subjected to reverse transcription reaction according to the product protocol of SuperScript III Reverse Transcriptase (Thermo Fisher Scientific (Life Technologies), 18080085). Quantitative PCR was carried out with real-time PCR system Aria MX (Agilent) according to the general manual of Brilliant III Ultra-Fast SYBR Green QPCR Master Mix (Agilent, 600882).

### Primers for PPR quantitative PCR

Fw: GATGAGGCTTTGGAACTGTTTG (SEQ ID NO. 26)
Rv: TCTCTGGCTCTGCCGGCCTTGC (SEQ ID NO. 27)

### Primers for GAPDH quantitative PCR

Fw: ATCATCCCTGCCTCTACTGG (SEQ ID NO. 28)
Rv: CTGCTTCACCACCTTCTTGA (SEQ ID NO. 29)

### <Results>

The results of quantitative PCR are shown in Figure 18. As a result of quantitative PCR, continued expression was confirmed even at Day 183 after administration, and a rise in time-dependent expression was also confirmed. It is shown in the preceding articles that expression can last for a long time (6 months or longer) when AAV9 is administered in the tail vein, and the results of the present experiment also agree with the said knowledge.

From the above, it was suggested that the present invention may exert the desired effect for a long time with fewer number of administrations to the subject.

## Claims

1. A pharmaceutical composition for treating myotonic dystrophy type 1, wherein
said pharmaceutical composition comprises a nucleic acid encoding a protein that specifically binds to the CUG repeat sequence,
said protein that specifically binds to the CUG repeat sequence comprises at least 6 pentatricopeptide repeat (PPR) motifs consisting of polypeptides of 30 - 38 amino acids long represented by Formula 1,
[Formula 1]
**(Helix A)-X-(Helix B)-L** **(Formula 1)**
[wherein in Formula 1:
Helix A is a 12 amino acids long moiety that is capable of forming an α-helix structure, and is represented by Formula 2, [Formula 2]
**A₁-A₂-A₃-A₄-A₅-A₆-A₇-A₈-A₉-A₁₀-A₁₁-A₁₂** **(Formula 2)**
wherein in Formula 2, A₁ - A₁₂ each independently represents an amino acid;
X is absent, or is a moiety consisting of a length of 1 - 9 amino acids;
Helix B is a moiety that is capable of forming an α-helix structure consisting of a length of 11 - 13 amino acids;
L is a moiety represented by Formula 3 that is 2 - 7 amino acids long;
[Formula 3]
**Lᵥᵢᵢ-Lᵥᵢ-Lᵥ-Lᵢᵥ-Lᵢᵢᵢ-Lᵢᵢ-Lᵢ** **(Formula 3)**
and wherein in Formula 3, each amino acid is numbered from the C-terminal such as "i" (-1), "ii" (-2),
provided that Lᵢᵢᵢ - Lᵥᵢᵢ may be absent]
and the combination of three amino acids at A₁, A₄, and Lᵢᵢ, or the combination of two amino acids at A₄ and Lᵢᵢ in said each PPR motif is selected so that said each PPR motif binds to C, U, or G, and thereby configured so that said protein specifically binds to said CUG repeat sequence.

2. The pharmaceutical composition according to claim 1, wherein said protein that specifically binds to the CUG repeat sequence comprises 9 - 30 of said PPR motifs.

3. The pharmaceutical composition according to claim 2, wherein said protein that specifically binds to the CUG repeat sequence comprises 12 - 24 of said PPR motifs.

4. The pharmaceutical composition according to any one of claim 1 to 3, wherein the combination of three amino acids at A₁, A₄, and Lᵢᵢ in said each PPR motif is:
when the base to be the target for the PPR motif is A (adenine), the combination of three amino acids at A₁, A₄, and Lᵢᵢ is, in the order of (A₁, A₄, Lii), (valine, threonine, asparagine), (phenylalanine, serine, asparagine), (phenylalanine, threonine, asparagine), (isoleucine, asparagine, aspartic acid), or (threonine, threonine, asparagine);
when the base to be the target for the PPR motif is G (guanine), the combination of three amino acids at A₁, A₄, and Lᵢᵢ is, in the order of (A₁, A₄, Lᵢᵢ), (glutamic acid, glycine, aspartic acid), (valine, threonine, aspartic acid), (lysine, threonine, aspartic acid), or (leucine, threonine, aspartic acid);
when the base to be the target for the PPR motif is U (uracil), the combination of three amino acids at A₁, A₄, and Lᵢᵢ is, in the order of (A₁, A₄, Lᵢᵢ), (valine, asparagine, aspartic acid), (isoleucine, asparagine, asparagine), (isoleucine, asparagine, aspartic acid), (isoleucine, methionine, aspartic acid), (phenylalanine, proline, aspartic acid), or (tyrosine, proline, aspartic acid); or
when the base to be the target for the PPR motif is C (cytosine), the combination of three amino acids at A₁, A₄, and Lᵢᵢ is, in the order of (A₁, A₄, Lᵢᵢ), (valine, asparagine, asparagine), (isoleucine, asparagine, asparagine), (valine, asparagine, serine), or (isoleucine, methionine, aspartic acid).

5. The pharmaceutical composition according to any one of claim 1 to 3, wherein the combination of two amino acids at A₄ and Lᵢᵢ in said each PPR motif is:
when the base to be the target for the PPR motif is A (adenine), the combination of two amino acids at A₄ and Lᵢᵢ is, in the order of (A₄, Lii), (threonine, asparagine), (serine, asparagine), or (glycine, asparagine);
when the base to be the target for the PPR motif is G (guanine), the combination of two amino acids at A₄ and Lᵢᵢ is, in the order of (A₄, Lii), (threonine, aspartic acid), or (glycine, aspartic acid);
when the base to be the target for the PPR motif is U (uracil), the combination of two amino acids at A₄ and Lᵢᵢ is, in the order of (A₄, Lii), (asparagine, aspartic acid), (proline, aspartic acid), (methionine, aspartic acid), or (valine, threonine);
when the base to be the target for the PPR motif is C (cytosine), the combination of two amino acids at A₄ and Lᵢᵢ is, in the order of (A₄, Lii), (asparagine, asparagine), (asparagine, serine), or (leucine, aspartic acid).

6. The pharmaceutical composition according to claim 1, **characterized in that** said nucleic acid encoding a protein that specifically binds to the CUG repeat sequence is integrated into an expression vector.

7. The pharmaceutical composition according to claim 1, **characterized in that** said nucleic acid encoding a protein that specifically binds to the CUG repeat sequence is integrated into a viral vector.

8. The pharmaceutical composition according to claim 7, **characterized in that** said viral vector is a viral vector with tropism towards muscle tissue.

9. The pharmaceutical composition according to claim 8, **characterized in that** said viral vector is an adeno-associated virus (AAV) vector, an adenovirus vector, a retrovirus vector, a lentivirus vector, or a herpes simplex virus vector.

10. The pharmaceutical composition according to claim 9, **characterized in that** said viral vector is an AAV vector.

11. The pharmaceutical composition according to claim 10, **characterized in that** said AAV vector is an AAV1 vector, an AAV2 vector, an AAV6 vector, an AAV7 vector, an AAV8 vector, an AAV9 vector, an AAV10 vector, an AAV11 vector, or an AAV12 vector.

12. An expression vector comprising the nucleic acid encoding the protein that specifically binds to the CUG repeat sequence as defined in claim 1.

13. A cell comprising the expression vector according to claim 12.

14. A protein that specifically binds to the CUG repeat sequence that is produced from the cell according to claim 13.

15. A viral expression vector comprising the nucleic acid encoding the protein that specifically binds to the CUG repeat sequence as defined in claim 1.

16. A cell comprising the viral expression vector according to claim 15.

17. A viral vector that is produced from the cell according to claim 16.

18. The use of the nucleic acid encoding the protein that specifically binds to the CUG repeat sequence as defined in claim 1 in the manufacture of myotonic dystrophy type 1 therapeutic medicine.

19. A method for treating myotonic dystrophy type 1, comprising a step of applying to a subject a therapeutically effective amount of the pharmaceutical composition according to claim 1.
